(19) Europäisches Patentamt
European Patent Office
Office européen des brevets

(11) Veröffentlichungsnummer: **0 409 025 A2**

(12) **EUROPÄISCHE PATENTANMELDUNG**

(21) Anmeldenummer: **90113021.1**

(22) Anmeldetag: **07.07.90**

(51) Int. Cl.5: **C07D 209/48**, A01N 43/38

(30) Priorität: **21.07.89 DE 3924052**

(43) Veröffentlichungstag der Anmeldung:
**23.01.91 Patentblatt 91/04**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Klausener, Alexander, Dr.**
**Dahlerdyk 173**
**D-4150 Krefeld 1(DE)**
Erfinder: **Wollweber, Detlef, Dr.**
**Paul-Ehrlich-Strasse 17**
**D-5600 Wuppertal 1(DE)**
Erfinder: **Seitz, Thomas, Dr.**
**Mozartstrasse 32**
**D-4019 Monheim(DE)**
Erfinder: **Kysela, Ernst, Dr.**
**Virchowstrasse 14**
**D-5060 Bergisch Gladbach 1(DE)**
Erfinder: **Lürssen, Klaus, Dr.**
**August-Kierspel-Strasse 145**
**D-5060 Bergisch Gladbach 2(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**

(54) **N-[Indol-6-yl]-heterocyclen.**

(57) Neue N-[Indol-6-yl]-heterocyclen der Formel (I),

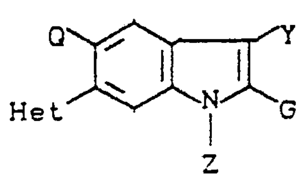

(I)

wobei Het für einen Heterocyclus steht,
mehrere Verfahren sowie neue Zwischenprodukte zu ihrer Herstellung und ihre Verwendung als Herbizide.

EP 0 409 025 A2

## N-[INDOL-6-YL]-HETEROCYCLEN

Die Erfindung betrifft neue N-[Indol-6-yl]-heterocyclen, ein Verfahren sowie neue Zwischenprodukte zu ihrer Herstellung und ihre Verwendung als Herbizide.

Es ist bereits bekannt, daß bestimmte N-[Indol-6-yl]-heterocyclen, wie beispielsweise 1-Isopropyl-5-fluorindol-6-yl-4,5,6,7-tetrahydro-2-H-isoindol-1,3-dione herbizide Eigenschaften besitzen (vgl. JP 63-174970).

Weiterhin ist die Synthese von N-[1-Methyl-indol-6-yl]-phthalimid bekannt (vgl. Doklady Akad. S.S.S.R. 118 [1958], 302, 305 und Pr. Acad. Sci. U.S.S.R. Chem. Sect. 118-123, [1958], 49, 51).

Es wurden nun neue N-[Indol-6-yl]-heterocyclen der allgemeinen Formel (I)

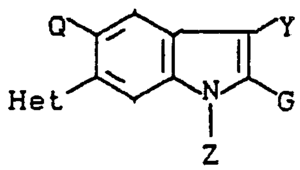

(I)

in welcher

Het für einen Heteracyclus der Formel

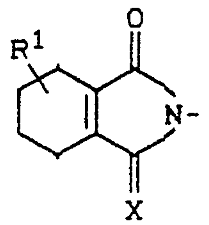 ; 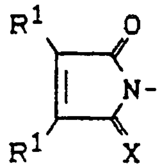 ; 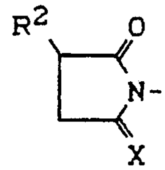 ;

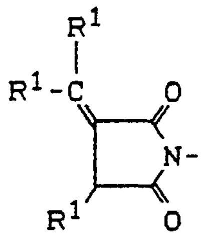 ; 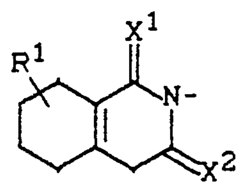 ; 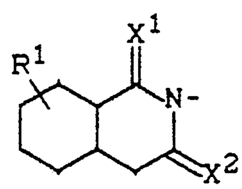 ;

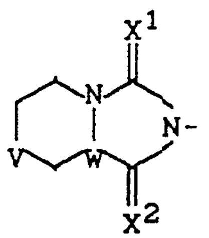 ; 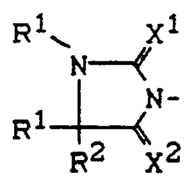 ; 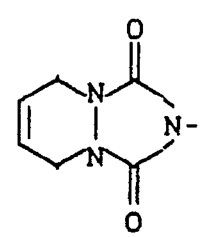 ;

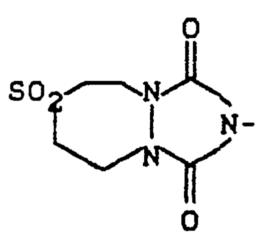 ; 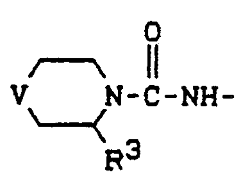 ; 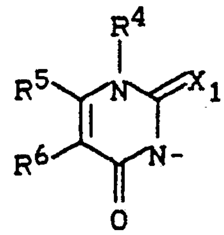 ;

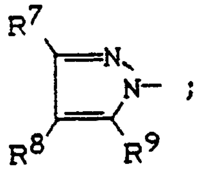
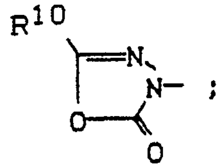
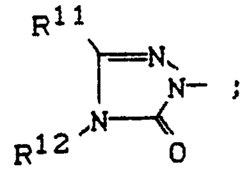

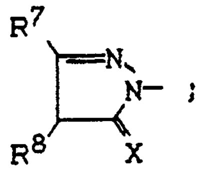
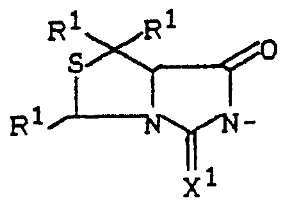
steht,

Q für Wasserstoff oder Halogen steht,
Y für Wasserstoff, Alkyl, vorzugsweise mit 1 bis 6 Kohlenstoffatomen, oder Halogen steht,
G für Wasserstoff oder Alkyl, vorzugsweise mit 1 bis 6 Kohlenstoffatomen, steht,
Z für Wasserstoff, unsubstituiertes oder substituiertes Alkyl, vorzugsweise mit 1 bis 10 Kohlenstoffatomen, unsubstituiertes oder substituiertes Alkenyl, vorzugsweise mit 2 bis 10 Kohlenstoffatomen, unsubstituiertes oder substituiertes Alkinyl, vorzugsweise mit 3 bis 10 Kahlenstoffatomen, unsubstituiertes oder substituiertes Cycloalkyl, vorzugsweise mit 3 bis 8 Kohlenstoffatomen, unsubstituiertes oder substituiertes Aralkyl, vorzugsweise mit 6 bis 10 Kohlenstoffatomen im Arylteil und 1 bis 6 Kohlenstoffatomen im Alkylteil, für Halogenalkyl, Alkoxycarbonyl oder (Di)alkylaminocarbonyl, vorzugsweise mit jeweils 1 bis 10 Kohlenstoffatomen in den einzelnen Alkylteilen, Alkylcarbonyl oder Alkylsulfonyl, vorzugsweise mit jeweils 1 bis 6 Kohlenstoffatomen, steht,
$R^1$ für Wasserstoff oder Alkyl mit 1 bis 8 Kohlenstoffatomen steht,
$R^2$ für Wasserstoff, Alkyl mit 1 bis 8 Kohlenstoffatomen oder Halogen steht,
$R^3$ für Cyano oder für den Rest $-COOR^1$ oder $-CONR^1R^1$ steht,
$R^4$, $R^5$ und $R^6$ entweder unabhängig voneinander jeweils für Wasserstoff oder Alkyl mit 1 bis 8 Kohlenstoffatomen stehen oder jeweils $R^4$ und $R^5$ gemeinsam oder $R^5$ und $R^6$ gemeinsam für eine Alkylkette mit 4 bis 6 Kohlenstoffatomen stehen,
$R^7$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^8$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^9$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halogenatomen oder Halogen steht
oder
$R^7$ und $R^8$ gemeinsam oder $R^8$ und $R^9$ gemeinsam für eine Alkylkette mit 4 bis 6 Kohlenstoffatomen stehen,
$R^{10}$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halogenatomen oder unsubstituiertes oder substituiertes Cycloalkyl mit 3 bis 7 Kohlenstoffatomen steht,
$R^{11}$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^{12}$ für Alkyl mit 1 bis 8 Kohlenstoffatomen oder Halogenalkyl mit 1 bis 8 Kohlenstoffatomen und 1 bis 17 gleichen oder verschiedenen Halogenatomen steht,
oder
$R^{11}$ und $R^{12}$ gemeinsam für eine Alkylkette mit 4 bis 6 Kohlenstoffatomen stehen,
V für Sauerstoff, Schwefel oder für eine Gruppe $-CH_2-$; $-SO_2-$ oder $-NCH_3$ steht,
W für Stickstoff oder die -CH-Gruppe steht,
X für Sauerstoff, Schwefel oder die $-CH_2$-Gruppe steht,
$X^1$ für Sauerstoff oder Schwefel steht und
$X^2$ für Sauerstoff oder Schwefel steht,
ausgenommen die Verbindungen N-[1-Methyl-indol-6-yl]-phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro-1H-isoindol-1,3(2H)-dion; 2-[5-Fluor-1-(1-methylethyl)-1H-indol-6-yl]-4,5,6,7-tetrahydro-1H-isoindol-1,3(2H)-dion, und 2-[5-Fluor-1-(1-methylpropyl)-1H-indol-6-yl]-4,5,6,7-tetrahydro-1H-isoindol-1,3(2H)-dion, 2-(5-Fluor-1H-indol-6-yl)-4,5,6,7-tetrahydro-1H-isoindol-1,3(2H)-dion (vgl. JP 63-174970, JP 63-313770, Doklady Akad. S.S.S.R. 118, [1958], 302, 305 und Pr. Acad. Sci. U.S.S.R. Chem. Sect. 118-123, [1958], 49, 51 (vgl. Beilstein, E III/IV 22 Seite 4297), gefunden.

Weiterhin wurde gefunden, daß man die neuen N-[Indol-6-yl]-heterocyclen der allgemeinen Formel (Ia)

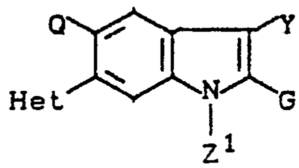

(Ia)

in welcher
$Z^1$ für die oben angegebenen Bedeutungen mit Ausnahme von Wasserstoff steht, Het, Q, G und Y die oben angegebenen Bedeutungen haben, ausgenommen die bereits oben genannten Verbindungen,
erhält, wenn man Heterocyclen der allgemeinen Formel (Ib)

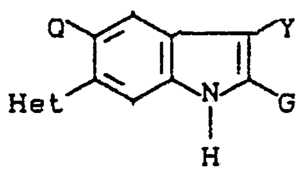

(Ib)

in welcher
Het, Q, Y und G die oben angegebene Bedeutung haben,

A) mit Alkylierungsmitteln der Formel (II)

$Z^1$-$E^1$ (II)

in welcher
$Z^1$ für unsubstituiertes oder substituiertes Alkyl, unsubstituiertes oder substituiertes Alkenyl, unsubstituiertes oder substituiertes Alkinyl, unsubstituiertes oder substituiertes Cycloalkyl, unsubstituiertes oder substituiertes Aralkyl oder Halogenalkyl steht und,
$E^1$ für eine elektronenanziehende Abgangsgruppe steht,
oder

B) mit Acylierungsmitteln der Formel (IIIa)

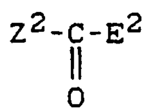

(IIIa)

in welcher
$Z^2$ für Alkoxy, (Di)alkylamino oder Alkyl steht und
$E^2$ für eine elektronenanziehende Abgangsgruppe steht,
oder

C) mit Sulfonylierungsmitteln der Formel (IIIb)

$Z^3$-$SO_2$-$E^3$ (IIIb)

in welcher
$Z^3$ für Alkyl steht und
$E^3$ für eine elektronenanziehende Abgangsgruppe steht,
jeweils gegebenenfalls in Gegenwart eines Verdünnungsmittels und gegebenenfalls in Gegenwart eines Reaktionshilfsmittels umsetzt.

Weiterhin wurde gefunden, daß man die neuen N-[Indol-6-yl]-heterocyclen der Formel (Ib), in denen Z für Wasserstoff steht, nach einem der im Folgenden beschriebenen Verfahren erhält:

D) Man erhält N-[Indol-6-yl]-heterocyclen der allgemeinen Formel (Ib-A)

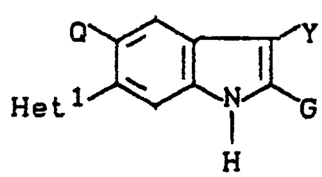

(Ib-A)

in welcher
Het[1] für einen Heterocyclus der Formel

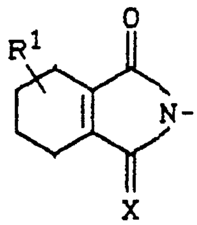 ; 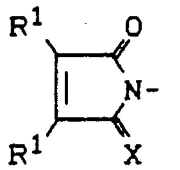 ; 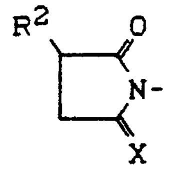 ;

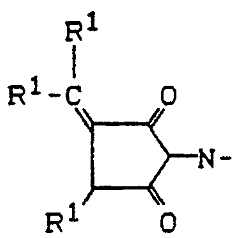 ; 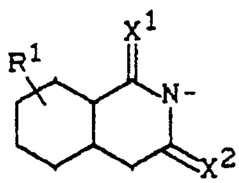 oder 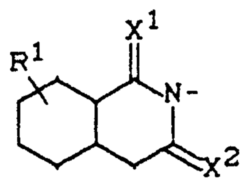

steht und
Q, Y, G, $R^1$, $R^2$, X, $X^1$ und $X^2$ die oben angegebene Bedeutung haben,
wenn man 6-Amino-indole der Formel (IV)

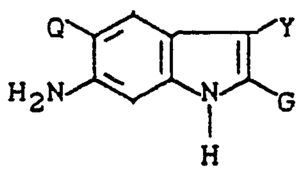

(IV)

in welcher
Q, Y und G die oben angegebene Bedeutung haben, mit Anhydriden der Formel (Va) - (Vf)

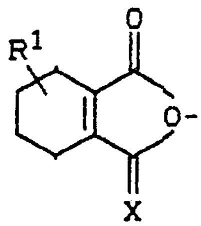 ;

(Va)

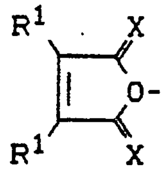 ;

(Vb)

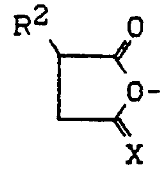 ;

(Vc)

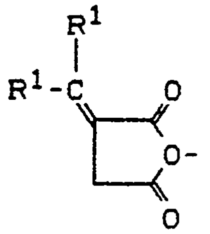 ;

(Vd)

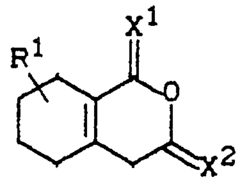 ;

(Ve)

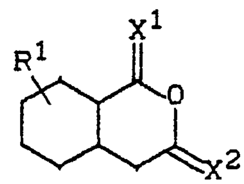

(Vf)

in welcher
$R^1$, $R^2$, X, $X^1$ und $X^2$ die oben angegebene Bedeutung haben,
gegebenenfalls in Gegenwart eines Verdünnungsmittels und gegebenenfalls in Gegenwart eines Reaktionshilfsmittels umsetzt;
oder
E) man erhält Heterocyclen der allgemeinen Formel (Ib-B)

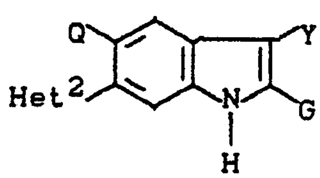

(Ib-B)

in welcher
$Het^2$ für einen Heterocyclus der Formel

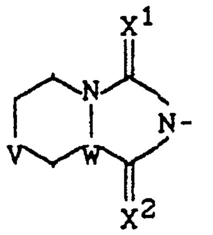 ; 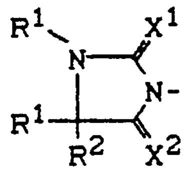 ; 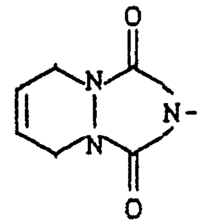 ;

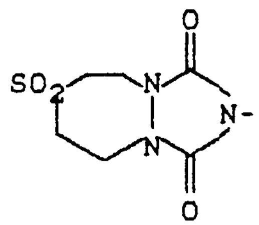 ; 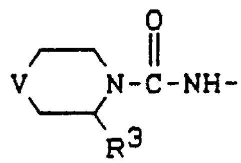 ; 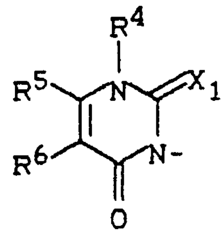 ;

oder 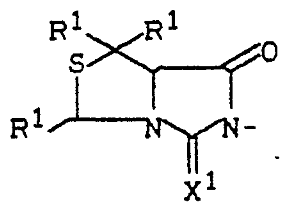

steht und

Q, Y, G, V, W, $R^1$, $R^2$, $R^3$, $R^4$, $R^5$, $R^6$, $X^1$ und $X^2$ die oben angegebene Bedeutung haben;

(E-α) wenn man Iso(thio)cyanate der Formel (VI)

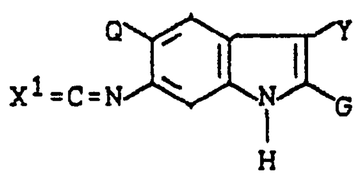

(VI)

in welcher

Q, Y und G die oben angegebene Bedeutung haben, oder

(E-β) wenn man Carbamate der Formel (VII)

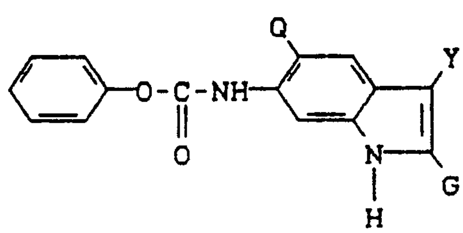

(VII)

in welcher

Q, Y und G die oben angegebene Bedeutung haben, mit Carbonsäureestern der Formel (VIIIa)

$R^{13}$-$COOR^{14}$ (VIIIa) in welcher

$R^{13}$ für einen Rest der Formel

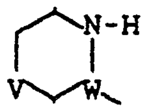 ; 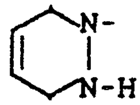 ; 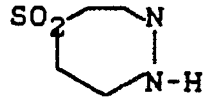 ;

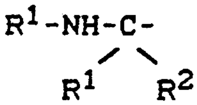 ; 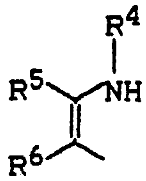 oder 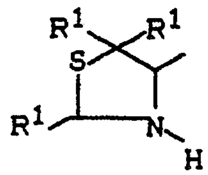 steht,

in welcher
$R^1$, $R^2$, $R^4$, $R^5$, $R^6$, V und W die oben angegebene Bedeutung haben und
$R^{14}$ für Alkyl steht,
oder mit Aminen der Formel (VIIIb)

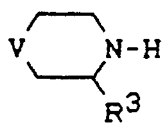 ; (VIIIb)

in welcher
V und $R^3$ die oben angegebene Bedeutung haben, oder deren Säureadditionssalze,
gegebenenfalls in Gegenwart eines Verdünnungsmittels und gegebenenfalls in Gegenwart eines Reaktionshilfsmittels umsetzt;
(F) man erhält Heterocyclen der allgemeinen Formel (Ib-C)

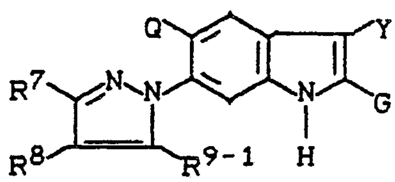 (Ib-C)

in welcher
$R^7$, $R^8$, Q, Y und G die oben angegebene Bedeutung haben und
$R^{9-1}$ für Wasserstoff, Alkyl oder Halogenalkyl steht,
wenn man Hydrazine der Formel (IX)

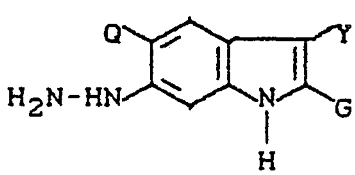 (IX)

in welcher
Q, Y und G die oben angegebene Bedeutung haben, mit 1,3-Diketonen der Formel (X)

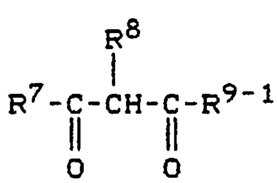 (X)

in welcher
$R^7$, $R^8$ und $R^{9-1}$ die oben angegebene Bedeutung haben,
oder mit Derivaten dieser Diketone, wie beispielsweise Enolethern, Enolestern, Ketalen, Enoletherketalen, Enaminen oder β-Halogenvinylketonen gegebenenfalls in Gegenwart eines Verdünnungsmittels und gegebenenfalls in Gegenwart eines Reaktionshilfsmittels umsetzt;
G) man erhält Heterocyclen der allgemeinen Formel (Ib-D)

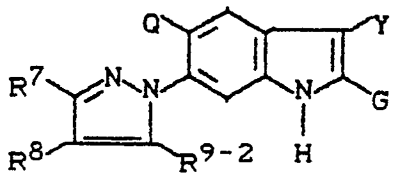

(Ib-D)

in welcher
$R^7$, $R^8$, Q, Y und G die oben angegebene Bedeutung haben und
$R^{9-2}$ für Halogen steht,
wenn man Pyrazolinyl-indole der Formel (XI)

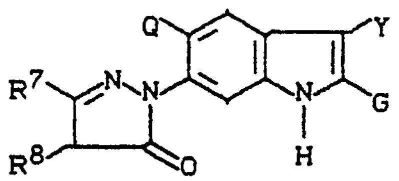

(XI)

in welcher
$R^7$, $R^8$, Q, Y und G die oben angegebene Bedeutung haben,
mit einem Halogenierungsmittel, gegebenenfalls in Gegenwart eines Verdünnungsmittels und gegebenenfalls in Gegenwart eines Reaktionshilfsmittels umsetzt;
H) man erhält Heterocyclen der allgemeinen Formel (Ib-E)

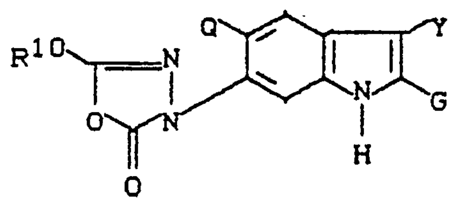

(Ib-E)

in welcher
$R^{10}$, Q, Y und G die oben angegebene Bedeutung haben,
wenn man Hydrazide der Formel

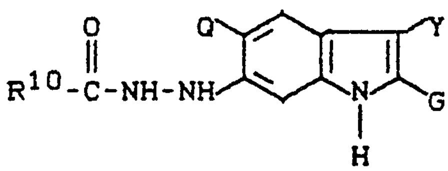

(XII)

in welcher
$R^{10}$, Q, Y und G die oben angegebene Bedeutung haben, mit Phosgen gegebenenfalls in Gegenwart eines Verdünnungsmittels und gegebenenfalls in Gegenwart eines Reaktionshilfsmittels umsetzt;
I) man erhält Heterocyclen der allgemeinen Formel (Ib-F)

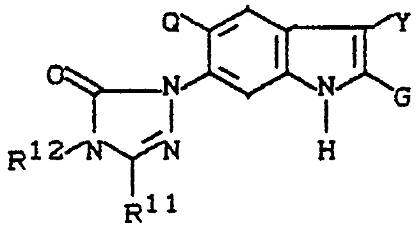

(Ib-F)

in welcher
$R^{11}$, $R^{12}$, Q, Y und G die oben angegebene Bedeutung haben,
wenn man Verbindungen der Formel (XIII)

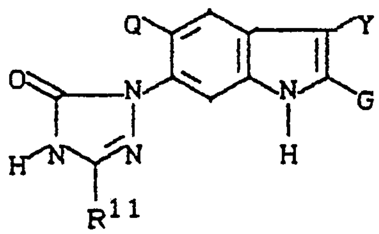

(XIII)

in welcher
$R^{11}$, Q, Y und G die oben angegebene Bedeutung haben, mit Alkylierungsmitteln der Formel (XIV)
$R^{12}$-E (XIV)
in welcher
$R^{12}$ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt;
K) man erhält Heterocyclen der allgemeinen Formel (Ib-G)

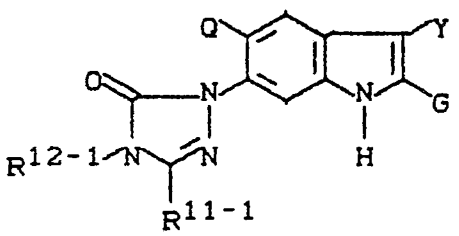

(Ib-G)

in welcher
Q, Y und G die oben angegebene Bedeutung haben und $R^{12-1}$ und $R^{11-1}$ gemeinsam für einen zweifach verknüpften Alkandiylrest stehen,
wenn man Amidrazone der Formel (XV)

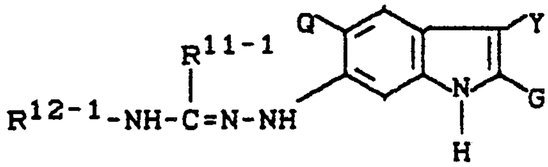

(XV)

in welcher
$R^{11-1}$ und $R^{12-1}$ die oben angegebene Bedeutung haben,
mit Phosgen gegebenenfalls in Gegenwart eines Verdünnungsmittels und gegebenenfalls in Gegenwart eines Reaktionshilfsmittels umsetzt;

L) man erhält Heterocyclen der allgemeinen Formel (Ib-H)

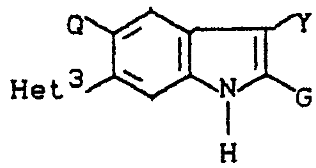

(Ib-H)

in welcher
Het³ für einen Heterocyclus der Formel

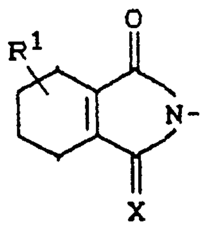 ; 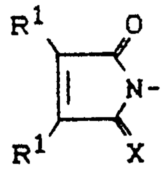 ; 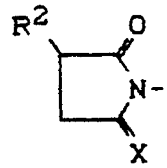 ;

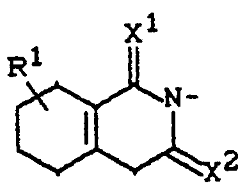 ; 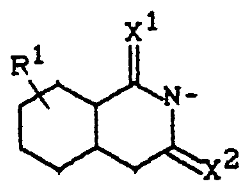 ; 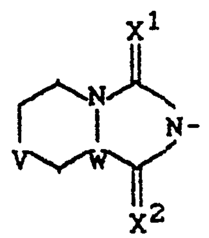 ;

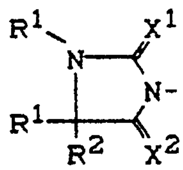 ; 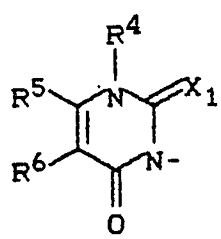 oder 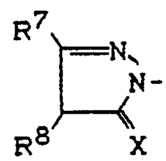

steht und
Q, Y, G, $R^1$, $R^2$, $R^4$, $R^5$, $R^6$, $R^7$, $R^8$, X, $X^1$, $X^2$, V und W die oben angegebene Bedeutung haben, wobei X für Schwefel und $X^1$ und $X^2$ jeweils für Sauerstoff oder Schwefel stehen, mit der Bedingung, daß mindestens einer der Reste $X^1$ oder $X^2$ für Schwefel steht;
(L-α) wenn man die mit Hilfe der Verfahren (D) und (E) erhältlichen Heterocyclen der Formel (Ib-I)

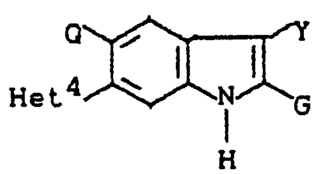

(Ib-I)

in welcher
$Het^4$ für einen Heterocyclus der Formel

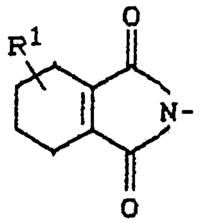 ; 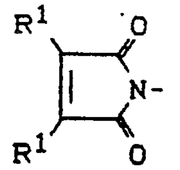 ; 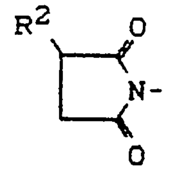 ;

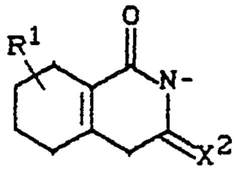 ; 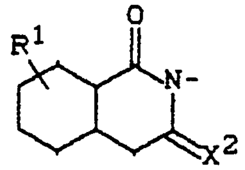 ; 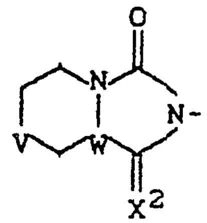 ;

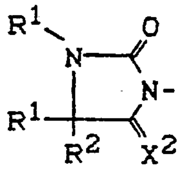 oder 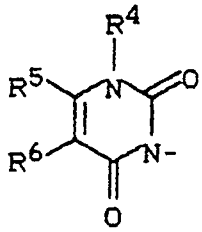

steht und
Q, Y, G, $R^1$, $R^2$, $R^4$, $R^5$, $R^6$, V, W und $X^2$ die oben angegebene Bedeutung haben;
oder
(L-β) wenn man Pyrazolinyl-indole der Formel (XI)

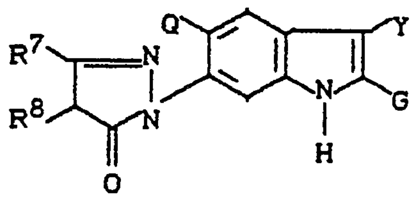

(XI)

in welcher
$R^7$, $R^8$, Q, Y und G die oben angegebene Bedeutung haben,
mit "Lawessons-Reagenz" der Formel (XVI)

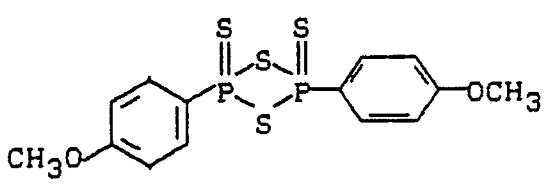

(XVI)

gegebenenfalls in Gegenwart eines Verdünnungsmittels umsetzt.

Schließlich wurde gefunden, daß die neuen N-[Indol-6-yl]-heterocyclen der allgemeinen Formel (I) herbizide Eigenschaften besitzen.

Überraschenderweise zeigen die erfindungsgemäßen N-[Indol-6-yl]-heterocyclen der allgemeinen Formel (I) eine hervorragende herbizide Wirksamkeit gegenüber wichtigen Problemunkräutern und gleichzeitig eine ausgezeichnete Verträglichkeit gegenüber wichtigen Kulturpflanzen.

Die erfindungsgemäßen N-(Indol-6-yl]-heterocyclen sind durch die Formel (I) allgemein definiert.

In den aliphatischen Resten wie beispielsweise Alkyl, Alkenyl, Alkinyl, Alkoxy oder Halogenalkyl, sind die Kohlenstoffketten jeweils geradkettig oder verzweigt.

Bevorzugt sind Verbindungen der Formel (I), bei welchen Het für einen Heterocyclus der Formel

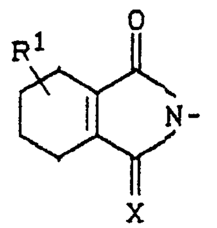 ; 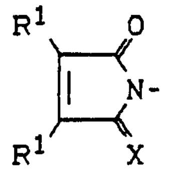 ; 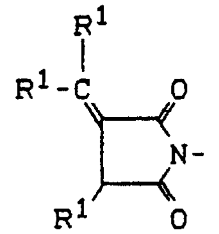 ;

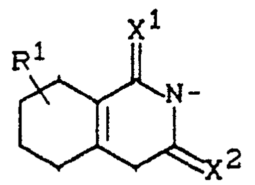 ; 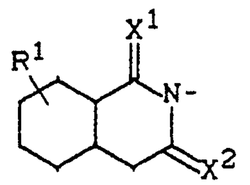 ; 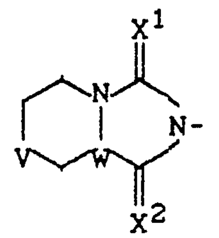 ;

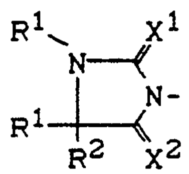 ; 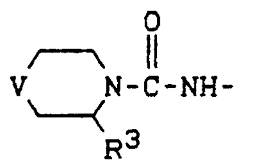 ; 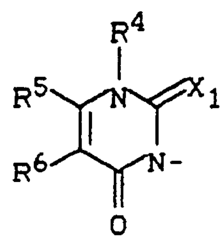 ;

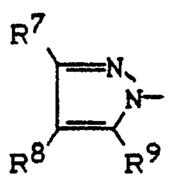 ; 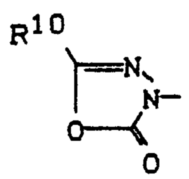 ; 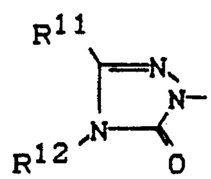 ;

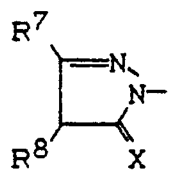 oder 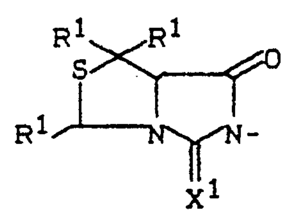

steht,

Q für Wasserstoff, Fluor, Chlor oder Brom steht,

Y für Wasserstoff, Alkyl mit 1 bis 4 Kohlenstoffatomen oder Fluor, Chlor oder Brom steht,

G für Wasserstoff oder Alkyl mit 1 bis 4 Kohlenstoffatomen steht,

Z für Wasserstoff, unsubstituiertes oder substituiertes Alkyl mit 1 bis 8 Kohlenstoffatomen, für unsubstituiertes oder substituiertes Alkenyl mit 2 bis 8 Kohlenstoffatomen, für unsubstituiertes oder substituiertes Alkinyl mit 3 bis 8 Kohlenstoffatomen, wobei als Substituenten jeweils Methoxy, Ethoxy, n- oder i-Propoxy, n-, i-, s- oder t-Butoxy, Cyano, Alkoxycarbonyl mit 1 bis 4 Kohlenstoffatomen oder Cyclohexyl mit 3 bis 7 Kohlenstoffatomen in Frage kommen; für unsubstituiertes oder einfach oder mehrfach, gleich oder verschieden substituiertes Cycloalkyl mit 3 bis 6 Kohlenstoffatomen, wobei als Substituenten Fluor, Chlor, Methyl, Ethyl, n- oder i-Propyl genannt seien; unsubstituiertes oder einfach oder mehrfach, gleich oder verschieden substituiertes Phenylalkyl mit 1 bis 4 Kohlenstoffatomen im Alkylteil, wobei als Substituenten in Frage kommen: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 oder 2 Kohlenstoffatomen und

1 bis 5 gleichen oder verschiedenen Halagenatomen;
Z steht weiterhin für Halogenalkyl mit 1 bis 8 Kohlenstoffatomen und 1 bis 17 gleichen oder verschiedenen Halogenatomen, Alkoxycarbonyl mit 1 bis 8 Kohlenstoffatomen im Alkoxyteil, (Di)alkylaminocarbonyl mit 1 bis 8 Kohlenstoffatomen in den einzelnen Alkylteilen, Acyl mit 1 bis 4 Kohlenstoffatomen oder Alkylsulfonyl mit 1 bis 4 Kohlenstoffatomen,
$R^1$ für Wasserstoff oder Alkyl mit 1 bis 4 Kohlenstoffatomen steht,
$R^2$ für Wasserstoff, Alkyl mit 1 bis 4 Kohlenstoffatomen, Fluor oder Chlor steht,
$R^3$ für Cyano oder für den Rest
$-COOR^1$ oder $-CONR^1R^1$
steht,
$R^4$, $R^5$ und $R^6$ entweder unabhängig voneinander jeweils für Wasserstoff oder Alkyl mit 1 bis 4 Kohlenstoffatomen stehen oder jeweils $R^4$ und $R^5$ gemeinsam oder $R^5$ und $R^6$ gemeinsam für eine Alkylkette mit 4 oder 5 Kohlenstoffatomen stehen,
$R^7$ für Wasserstoff, Alkyl mit 1 bis 4 Kohlenstoffatomen oder Halogenalkyl mit 1 bis 4 Kohlenstoffatomen und 1 bis 9 gleichen oder verschiedenen Halogenatomen steht,
$R^8$ für Wasserstoff, Alkyl mit 1 bis 4 Kohlenstoffatomen oder Halogenalkyl mit 1 bis 4 Kohlenstoffatomen und 1 bis 9 gleichen oder verschiedenen Halogenatomen steht,
$R^9$ für Wasserstoff, Fluor, Chlor, Brom, Alkyl mit 1 bis 4 Kohlenstoffatomen oder Halogenalkyl mit 1 bis 4 Kohlenstoffatomen und 1 bis 9 gleichen oder verschiedenen Halogenatomen steht oder
$R^7$ und $R^8$ gemeinsam oder $R^8$ und $R^9$ gemeinsam für eine Alkylkette mit 4 oder 5 Kohlenstoffatomen stehen,
$R^{10}$ für Wasserstoff, Alkyl mit 1 bis 4 Kohlenstoffatomen, Halogenalkyl mit 1 bis 4 Kohlenstoffatomen und 1 bis 9 gleichen oder verschiedenen Halogenatomen oder unsubstituiertes oder einfach bis dreifach, gleich oder verschieden durch Methyl, Ethyl, n-oder i-Propyl, Fluor oder Chlor substituiertes Cycloalkyl mit 3 bis 5 Kohlenstoffatomen steht,
$R^{11}$ für Wasserstoff, Alkyl mit 1 bis 4 Kohlenstoffatomen oder Halogenalkyl mit 1 bis 4 Kohlenstoffatomen und 1 bis 9 gleichen oder verschiedenen Halogenatomen steht,
$R^{12}$ für Alkyl mit 1 bis 4 Kohlenstoffatomen oder Halogenalkyl mit 1 bis 4 Kohlenstoffatomen und 1 bis 9 gleichen oder verschiedenen Halogenatomen steht, oder
$R^{11}$ und $R^{12}$ gemeinsam für eine Alkylkette mit 4 oder 5 Kohlenstoffatomen steht,
V für Sauerstoff, Schwefel oder für eine Gruppe $-CH_2-$; $SO_2$ oder $-NCH_3$ steht,
W für Stickstoff oder die -CH-Gruppe steht,
X für Sauerstoff, Schwefel oder die $-CH_2$-Gruppe steht,
$X^1$ für Sauerstoff oder Schwefel steht und
$X^2$ für Sauerstoff oder Schwefel steht,
ausgenommen die Verbindungen N-[1-Methyl-indol-6-yl]-phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro-1H-isoindol-1,3(2H)-dion; 2-[5-Fluor-1-(1-methylethyl)-1H-indol-6-yl]-4,5,6,7-tetrahydro-1H-isoindol-1,3(2H)-dion, 2-[5-Fluor-1-(1-methylpropyl)-1H-indol-6-yl]-4,5,6,7-tetrahydro-1H-isoindol-1,3(2H)-dion und 2-(5-Fluor-1H-indol-6-yl)-4,5,6,7-tetrahydro-1H-isoindol-1,3(2H)-dion.

Besonders bevorzugt ist die Gruppe von Verbindungen der Formel (I), bei welchen
Het für einen Heterocyclus der Formel

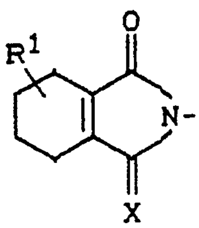 ; 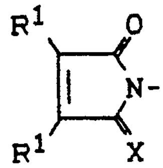 ; 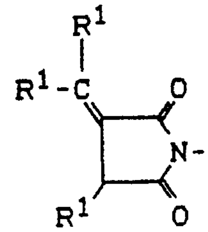 ;

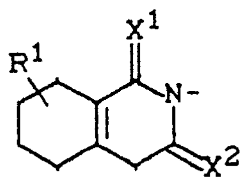 ; 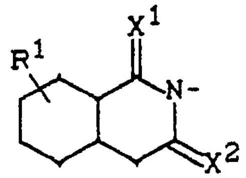 ; 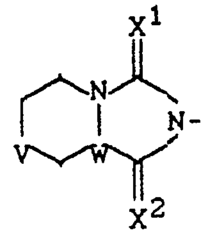 ;

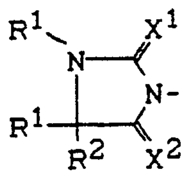 ; 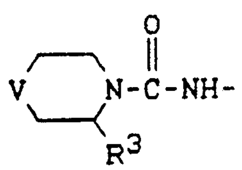 ; 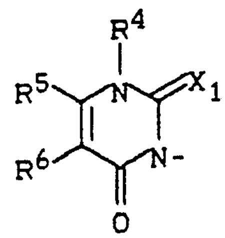 ;

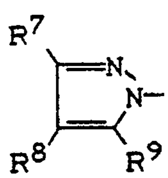 ; 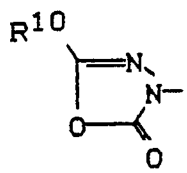 ; 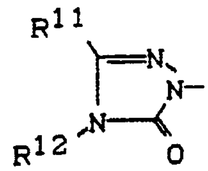 oder

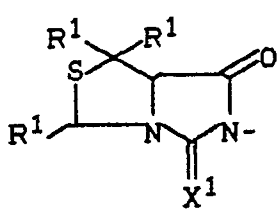 steht,

Q für Wasserstoff, Fluor oder Chlor steht,

Y für Wasserstoff, Methyl, Ethyl, n- oder i-Propyl, n-, i-, s- oder t-Butyl, Brom oder Chlor steht,

G für Wasserstoff, Methyl, Ethyl, n- oder i-Propyl, n-, i-, s- oder t-Butyl steht,

Z für unsubstituiertes oder substituiertes Alkyl mit 1 bis 6 Kohlenstoffatamen, für unsubstituiertes oder substituiertes Alkenyl mit 2 bis 6 Kohlenstoffatomen, für unsubstituiertes oder substituiertes Alkinyl mit 3 bis 6 Kohlenstoffatomen, wobei als Substituenten jeweils Methoxy, Ethoxy, n- oder i-Propoxy, n-, i-, s- oder t-Butoxy, Cyano, Alkoxycarbonyl mit 1 bis 4 Kohlenstoffatomen oder Cyclohexyl mit 3 bis 6 Kohlenstoffatomen infrage kommen; für unsubstituiertes oder einfach bis dreifach, gleich oder verschieden substituiertes Cyclopentyl oder Cyclohexyl, wobei als Substituenten Fluor, Chlor oder Methyl genannt seien; jeweils unsubstituiertes oder einfach bis dreifach, gleich oder verschieden substituiertes Benzyl oder Phenethyl, wobei als Substituenten infrage kommen: Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy oder Trifluormethylthio;

Z steht weiterhin für Halogenalkyl mit 1 bis 4 Kohlenstoffatomen und 1 bis 9 Fluor und/oder Chloratomen, Alkoxycarbonyl mit 1 bis 4 Kohlenstoffatomen im Alkoxyteil, (Di)alkylaminocarbonyl mit 1 bis 4 Kohlenstoffatomen in den einzelnen Alkylteilen, Acetyl, Propionyl, Methansulfonyl und Ethansulfonyl,

$R^1$ für Wasserstoff, Methyl, Ethyl, n- oder i-Propyl oder n-, i-, s- oder t-Butyl steht,

$R^2$ für Wasserstoff, Methyl, Ethyl, n- oder i-Propyl, n-, i-, s- oder t-Butyl steht,

$R^3$ für Cyano oder für den Rest $-COOR^1$ oder $-CONR^1R^1$ steht,

$R^4$, $R^5$ und $R^6$ entweder unabhängig voneinander jeweils für Wasserstoff, Methyl, Ethyl, n- oder i-Propyl, n-, i-, s- oder t-Butyl stehen oder jeweils $R^4$ und $R^5$ gemeinsam oder $R^5$ und $R^6$ gemeinsam für eine Alkylkette mit 5 Kohlenstoffatomen stehen,

$R^7$ für Wasserstoff, Methyl, Ethyl, n- oder i-Propyl, n-, i-, s- oder t-Butyl oder Halogenalkyl mit 1 bis 4 Kohlenstoffatomen und 1 bis 9 Fluor- und/oder Chloratomen steht,

$R^8$ für Wasserstoff, Methyl, Ethyl, n- oder i-Propyl, n-, i-, s- oder t-Butyl oder Halogenmethyl mit 1 bis 3 Fluor- und/oder Chloratomen steht,

$R^9$ für Wasserstoff, Chlor, Methyl, Ethyl, n- oder i-Propyl, n-, i-, s- oder t-Butyl, Halogenalkyl mit 1 bis 2 Kohlenstoffatomen und 1 bis 5 Fluor-und/oder Chloratomen steht oder

$R^7$ und $R^8$ gemeinsam oder $R^8$ und $R^9$ gemeinsam für eine Alkylkette mit 5 Kohlenstoffatomen stehen,

$R^{10}$ für Wasserstoff, Methyl, Ethyl, n- oder i-Propyl, n-, i-, s- oder t-Butyl, Halogenalkyl mit 1 bis 4 Kohlenstoffatomen und 1 bis 9 Fluor- und/oder Chloratomen oder unsubstituiertes oder einfach bis dreifach gleich oder verschieden durch Methyl, Fluor und/oder Chlor substituiertes Cycloalkyl mit 3 bis 5 Kohlenstoffatomen steht,

$R^{11}$ für Wasserstoff, Methyl, Ethyl, n- oder i-Propyl, n-, i-, s- oder t-Butyl oder Halogenalkyl mit 1 bis 2 Kohlenstoffatomen und 1 bis 5 Fluor- und/oder Chloratomen steht,

$R^{12}$ für Methyl, Ethyl, n- oder i-Propyl, n-, i-, s-oder t-Butyl oder Halogenalkyl mit 1 bis 2 Kohlenstoffatomen und 1 bis 5 Fluor- und/oder Chloratomen steht,

oder

$R^{11}$ und $R^{12}$ gemeinsam für eine Alkylkette mit 5 Kohlenstoffatomen steht,

V für eine Gruppe $-CH_2-$; $SO_2$- oder $-NCH_3$ steht,

W für Stickstoff oder die -CH-Gruppe steht,

X für Sauerstoff, Schwefel oder die $-CH_2$-Gruppe steht,

$X^1$ für Sauerstoff oder Schwefel steht und

$X^2$ für Sauerstoff oder Schwefel steht.

Besonders bevorzugt ist auch die Gruppe von Verbindungen der Formel (I), in denen Z für Wasserstoff steht und die übrigen Substituenten die oben als besonders bevorzugt angegebenen Bedeutungen haben.

Ganz besonders bevorzugt ist die Gruppe von Verbindungen der Formel (I), bei welchen

Het für einen Heterocyclus der Formel

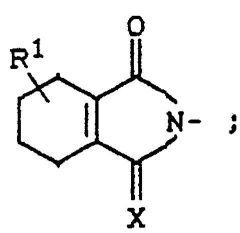

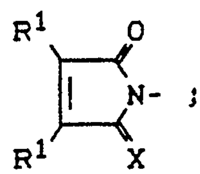

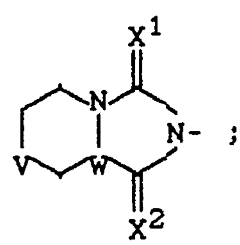

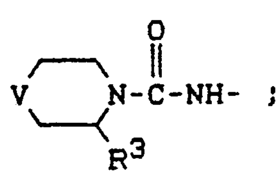

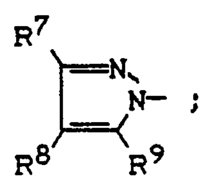

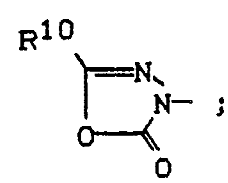

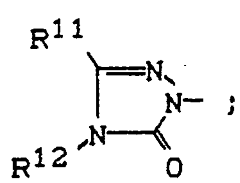

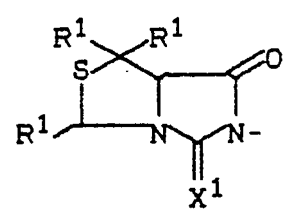

steht,

Q für Wasserstoff, Chlor oder Fluor steht,

Y für Wasserstoff, Methyl, Ethyl oder n- oder i-Propyl steht,
G für Wasserstoff, Methyl, Ethyl oder n- oder i-Propyl steht,
Z für Methyl, Ethyl, n- oder i-Propyl, n-Butyl, Methoxymethyl, Ethoxymethyl, Methoxyethyl, Ethoxyethyl, Propoxyethyl, Methoxycarbonyl, Ethoxycarbonyl, n- oder i-Propoxycarbonyl, Butoxycarbonyl, Cyanomethyl, Cyanoethyl, Cyclohexylmethyl, Propionitril-2-yl, Allyl, 2-Butenyl, Isopropenyl, Propargyl, 2-Butinyl, 3-Butin-2-yl, Trifluormethyl, 2,2,2-Trifluorethyl, 2-Fluorethyl-1-fluormethyl, Methoxycarbonyl, Ethoxycarbonyl, n-Propoxycarbonyl-i-propoxycarbonyl, Methylaminocarbonyl, Ethylaminocarbonyl, Dimethylaminocarbonyl oder Diethylaminocarbonyl steht,
$R^1$ für Wasserstoff, Methyl, Ethyl, n- oder i-Propyl steht,
$R^3$ für Cyano oder für den Rest
$-COOR^1$ oder $-CONR^1R^1$ steht,
$R^7$ für Wasserstoff, Methyl, Ethyl, n- oder i-Propyl, n-, i-, s- oder t-Butyl, Dichlorfluormethyl, Difluorchlormethyl oder Trifluormethyl steht,
$R^8$ für Wasserstoff, Methyl, Ethyl, n- oder i-Propyl, n-, i-, s- oder t-Butyl, Trifluormethyl, Chlordifluormethyl, Fluordichlormethyl, Chlor, Brom oder Nitro steht,
$R^9$ für Wasserstoff, Methyl, Ethyl, n- oder i-Propyl, n-, i-, s- oder t-Butyl, Trifluormethyl, Chlordifluormethyl, Fluordichlormethyl oder Chlor steht, oder
$R^7$ und $R^8$ gemeinsam oder $R^8$ und $R^9$ gemeinsam für eine Alkylkette mit 5 Kohlenstoffatomen stehen,
$R^{10}$ für Wasserstoff, Methyl, Ethyl, n- oder i-Propyl, t-Butyl, 1,3-Difluor-2-methyl-propyl-2-yl oder 1,2-Difluor-propyl-2-yl, Cyclopropyl oder einfach bis dreifach, gleich oder verschieden durch Methyl, Fluor und/oder Chlor substituiertes Cyclopropyl steht,
$R^{11}$ für Wasserstoff, Methyl, Ethyl, n- oder i-Propyl steht,
$R^{12}$ für Wasserstoff, Methyl, Ethyl, n- oder i-Propyl, Trifluormethyl, Chlordifluormethyl, Fluordichlormethyl, 1,1-Difluor-2,2-difluorethyl, 2,2,2-Trifluorethyl steht,
oder
$R^{11}$ und $R^{12}$ gemeinsam für eine Alkylkette mit 5 Kohlenstoffatomen steht,
V für die $-CH_2$-Gruppe steht,
W für Stickstoff oder die -CH-Gruppe steht,
X für Sauerstoff oder Schwefel steht,
$X^1$ für Sauerstoff oder Schwefel steht und $X^2$ für Sauerstoff oder Schwefel steht,
ausgenommen die Verbindungen N-[1-Methyl-indol-6-yl]phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro-1H-isoindol-1,3(2H)-dion; 2-[5-Fluor-1-(1-methylethyl)-1H-indol-6-yl]-4,5,6,7-tetrahydro-1H-isoindol-1,3(2H)-dion, 2-[5-Fluor-1-(1-methylpropyl)-1H-indol-6-yl]-4,5,6,7-tetrahydro-1H-isoindol-1,3(2H)-dion und 2-(5-Fluor-1H-indol-6-yl)-4,5,6,7-tetrahydro-1H-isoindol-1,3(2H)-dion.

Ganz besonders bevorzugt ist auch die Gruppe von Verbindungen der Formel (I), in denen Z für Wasserstoff steht und die übrigen Substituenten die oben als ganz besonders bevorzugt angegebenen Bedeutungen haben.

Für die Verbindungen der Formel (Ia) und (Ib) sei besonders die Bedeutung von

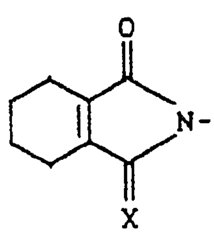

für Het hervorgehoben.

Inbesondere hervorgehoben sind die Verbindungen der Formel (I), in denen
Het für

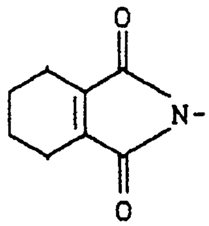

steht,
Q für Wasserstoff, Fluor oder Chlor steht,
Y für Wasserstoff, Methyl oder Ethyl steht,
G für Wasserstoff, Methyl oder Ethyl steht,
Z für Wasserstoff, Methyl, Ethyl, n- oder iso-Propyl, Allyl, Propargyl, Methoxycarbonylmethyl oder Methoxycarbonylethyl steht,
ausgenommen die Verbindungen N-[1-Methyl-indol-6-yl]-phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro-1H-isoindol-1,3(2H)-dion, 2-[5-Fluor-1-(1-methylethyl)-1H-indol-6-yl]-4,5,6,7-tetrahydro-1H-isoindol-1,3(2H)-dion und 2-(5-Fluor-1H-indol-6-yl)-4,5,6,7-tetrahydro-1H-isoindol-1,3(2H)-dion.

Im einzelnen seien außer den bei den Herstellungsbeispielen genannten Verbindungen die folgenden N-[Indol-6-yl]-heterocyclen der allgemeinen Formel (I) genannt:

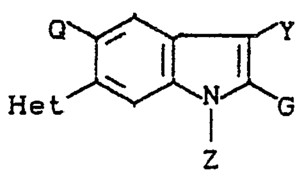

(I)

Tabelle 1

| Het | Q | Y | G | Z |
|---|---|---|---|---|
| 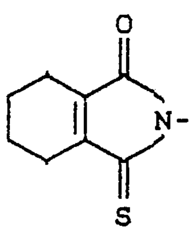 | F | H | H | $CH(CH_3)_2$ |
| 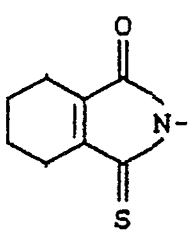 | F | H | H | $CH_2-C\equiv CH$ |

Tabelle 1 - Fortsetzung

| Het | Q | Y | G | Z |
|---|---|---|---|---|
| 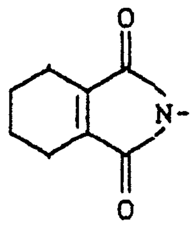 | H | $CH_3$ | H | $CH(CH_3)_2$ |
| 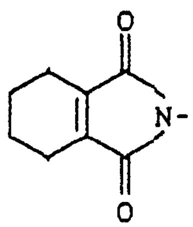 | Cl | H | $CH_3$ | $CH_2CH{=}CH_2$ |
| 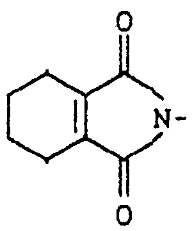 | F | $CH_3$ | H | $C_2H_5$ |
| 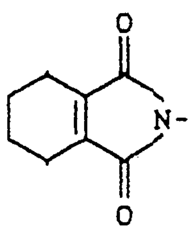 | F | H | H | $CO{-}C_2H_5$ |
| 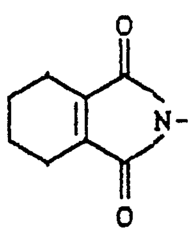 | F | H | H | $CO{-}N(CH_3)_2$ |
| 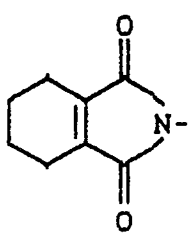 | F | H | H | $CH_2CN$ |

Tabelle 1 - Fortsetzung

| Het | Q | Y | G | Z |
|---|---|---|---|---|
| 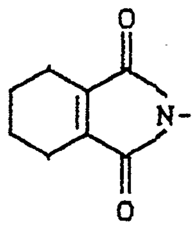 | F | H | H | $CF_2$-$CF_2H$ |
| 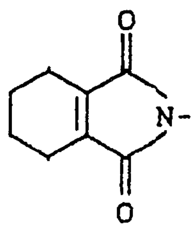 | F | H | H | CH-C≡CH<br>\|<br>$CH_3$ |
| 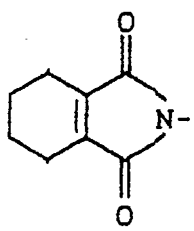 | F | H | $CH_3$ | $CH_2$-C≡CH |
| 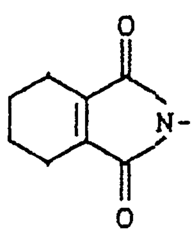 | F | $CH_3$ | H | $CH(CH_3)_2$ |
| 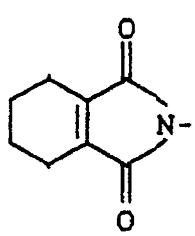 | F | $CH_3$ | H | n-$C_4H_9$ |
| 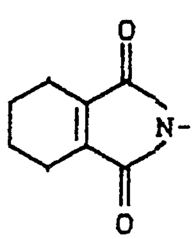 | F | H | $CH_3$ | $CH_2$-CH=CH-$CH_3$ |

Tabelle 1 - Fortsetzung

| Het | Q | Y | G | Z |
|---|---|---|---|---|
| 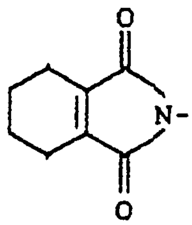 | F | $CH_3$ | H | $CH(CH_2F)_2$ |
| 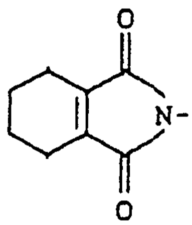 | F | Cl | H | $CH_3$ |
| 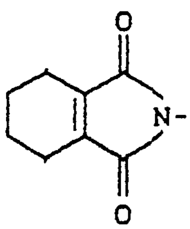 | F | Cl | H | $CH_2CN$ |
| 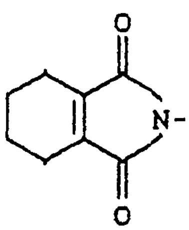 | H | Br | H | $CH_2COOC_2H_5$ |
| 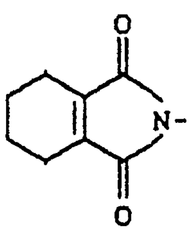 | H | H | H | $\underset{\underset{O}{\parallel}}{C}-OCH(CH_3)_2$ |

Tabelle 1 - Fortsetzung

| Het | Q | Y | G | Z |
|---|---|---|---|---|
| 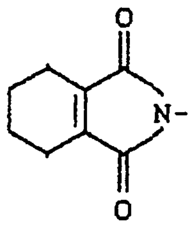 | F | Br | $CH_3$ | $CH_2$ 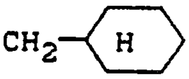 |
| 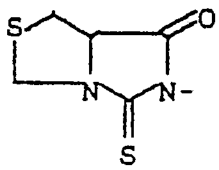 | F | H | H | $CH_2CH_2CH_2CH_3$ |
| 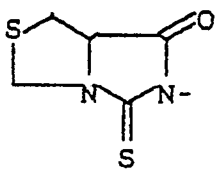 | F | H | H | $CH_2COOCH(CH_3)_2$ |
| 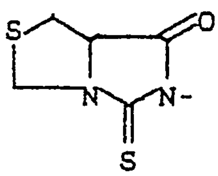 | F | H | H | $CH_2CH_2CH_2COOCH_3$ |
| 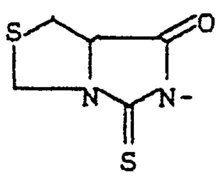 | Cl | H | H | $CH_2COOCH_3$ |
| 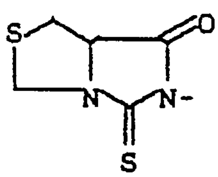 | Cl | H | H | $CH_2C{\equiv}CH$ |
| 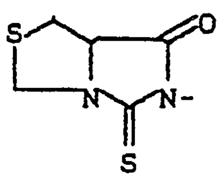 | H | H | H | $CH_2-CH=CCl_2$ |

Tabelle 1 - Fortsetzung

| Het | Q | Y | G | Z |
|---|---|---|---|---|
| 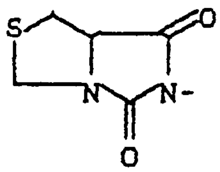 | F | H | H | $CH(CH_3)COOCH_3$ |
| 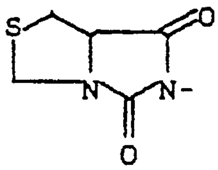 | F | H | H | $CH_2-C{\equiv}N$ |
| 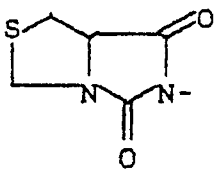 | F | H | H | $CH(CH_3)-C{\equiv}CH$ |
| 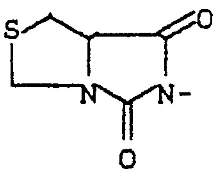 | F | $CH_3$ | $CH_3$ | $CH_2-C{\equiv}CH$ |
| 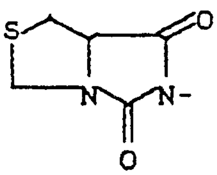 | Cl | H | H | $CH_2CH_2CH_2CH_3$ |
| 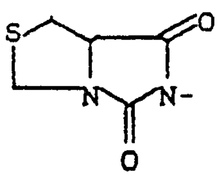 | Cl | H | H | $CH_2-CH=CH_2$ |
| 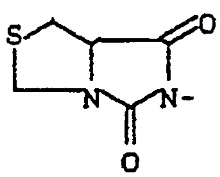 | Cl | $CH_3$ | H | $CH_2-CH=CH_2$ |

Tabelle 1 - Fortsetzung

| Het | Q | Y | G | Z |
|---|---|---|---|---|
| 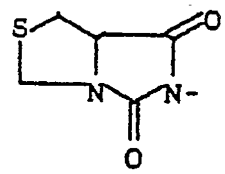 | H | H | H | $CH_2-C\equiv CH$ |
| 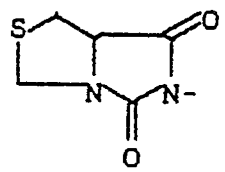 | H | H | $CH_3$ | $CH_2-C\equiv C-CH_3$ |
| 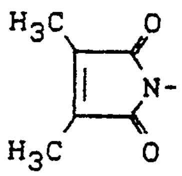 | F | H | H | $-CH_2C\equiv CH$ |
| 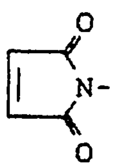 | F | H | H | $-CH_2C\equiv CH$ |
| 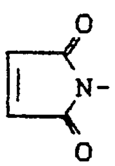 | F | $CH_3$ | H | $-CH_2C\equiv CH$ |
| 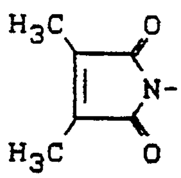 | Cl | H | H | $-CH_2CH=CH_2$ |
| 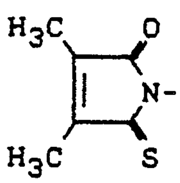 | F | H | $CH_3$ | $C_2H_5$ |

Tabelle 1 - Fortsetzung

| Het | Q | Y | G | Z |
|---|---|---|---|---|
| 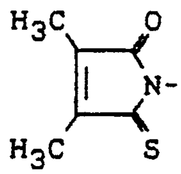 | Cl | H | H | $n-C_4H_9$ |
| 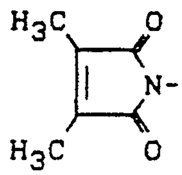 | F | H | H | $-CH(CH_3)_2$ |
| 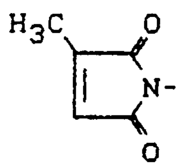 | F | H | H | $-CH_2C{\equiv}CH$ |
| 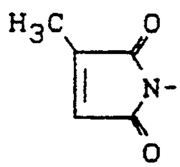 | H | $CH_3$ | H | $n-C_3H_7$ |
| 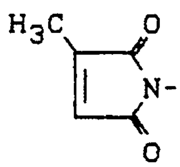 | Cl | H | H | $-CH_2C{\equiv}CH$ |
| 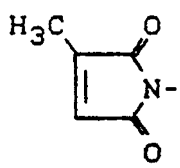 | F | H | $CH_3$ | $-CH_2CH_2-OCH_3$ |
| 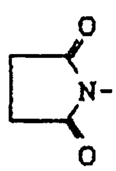 | F | H | H | $-CH_2-CH{=}CH_2$ |

Tabelle 1 - Fortsetzung

| Het | Q | Y | G | Z |
|---|---|---|---|---|
| 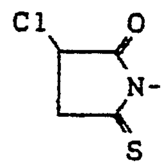 | F | H | H | $-CH_2-CH=CH_2$ |
| 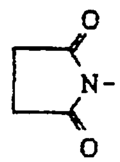 | F | H | H | $-CH_2-C≡CH$ |
| 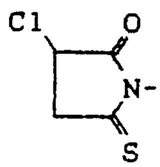 | F | H | H | $-CH_2-C≡CH$ |
| 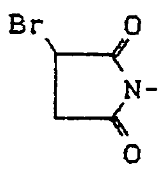 | F | H | H | $-CH_2-C≡C-CH_3$ |
| 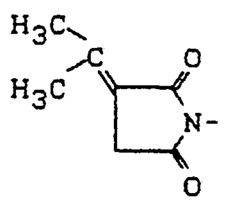 | F | $CH_3$ | H | $-CH_2-C≡CH$ |
| 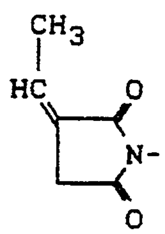 | F | H | H | $-CH_2-C≡CH$ |
| 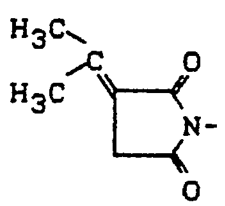 | F | Cl | H | $-CH(CH_3)_2$ |

Tabelle 1 - Fortsetzung

| Het | Q | Y | G | Z |
|---|---|---|---|---|
| 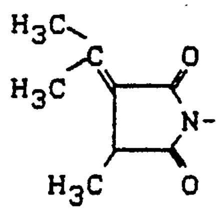 | H | H | $CH_3$ | $-CH_2-CH(CH_3)_2$ |
| 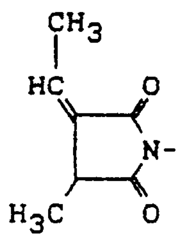 | H | H | H | 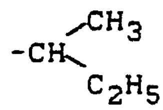 |
| 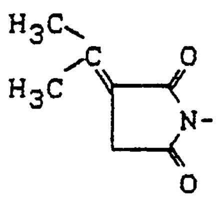 | H | H | H | 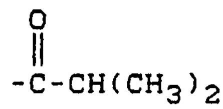 |
| 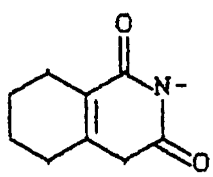 | F | H | H | $-CH_2C{\equiv}CH$ |
| 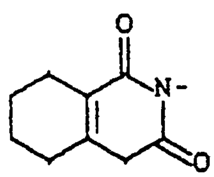 | F | H | $CH_3$ | $-CH_2-C{\equiv}CH$ |
| 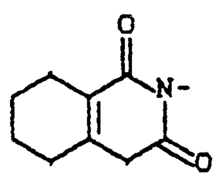 | F | H | H | $-CH(CH_3)_2$ |

Tabelle 1 - Fortsetzung

| Het | Q | Y | G | Z |
|---|---|---|---|---|
| 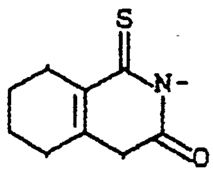 | F | H | H | $-C_2H_5$ |
| 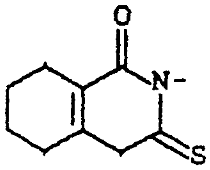 | F | H | H | $n-C_3H_7$ |
| 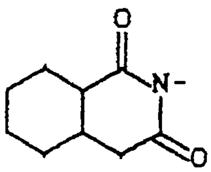 | F | H | H | $-CH_2C{\equiv}CH$ |
| 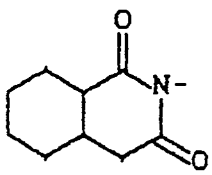 | F | H | H | $-CH_2-CN$ |
| 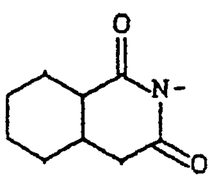 | F | H | H | $-CH(CH_3)_2$ |
| 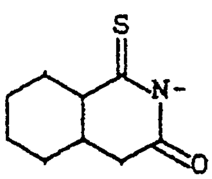 | F | H | H | $-C_2H_5$ |
| 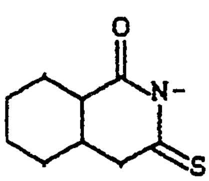 | F | H | H | $-CH_2CH_2OCH_3$ |

Tabelle 1 - Fortsetzung

| Het | Q | Y | G | Z |
|---|---|---|---|---|
| 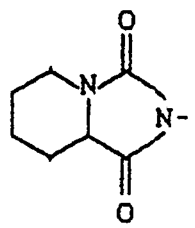 | F | H | H | $CH_2CN$ |
| 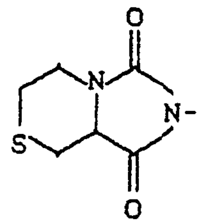 | F | H | H | $-CON(CH_3)_2$ |
| 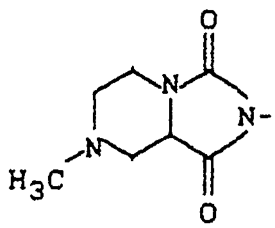 | F | H | H | $-CH_2-CH=CH_2$ |
| 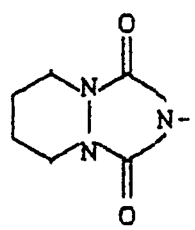 | F | H | H | $-CH_2-CH_2-OC_2H_5$ |
| 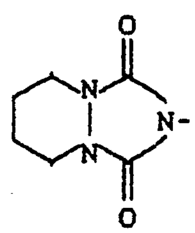 | F | H | H | $-CH_2CF_3$ |

Tabelle 1 - Fortsetzung

| Het | Q | Y | G | Z |
|---|---|---|---|---|
| 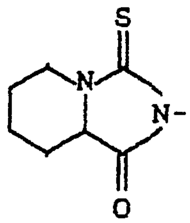 | F | H | H | $-CH_2CH(CH_3)_2$ |
| 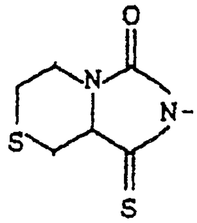 | F | H | H | $-CH_2-C(CH_3)_3$ |
| 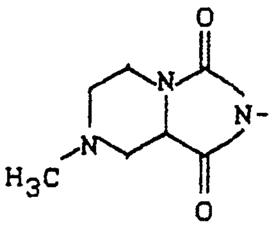 | F | H | H | $-CH_2-C{\equiv}CH$ |
| 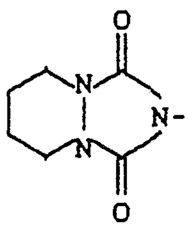 | F | H | H | $-CH_2-C{\equiv}CH$ |
| 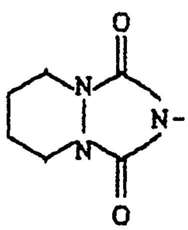 | F | H | H | $-CH_2-C{\equiv}CH$ |

Tabelle 1 - Fortsetzung

| Het | Q | Y | G | Z |
|---|---|---|---|---|
| 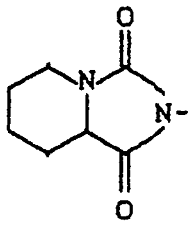 | F | H | H | $-CH_2-C{\equiv}CH$ |
| 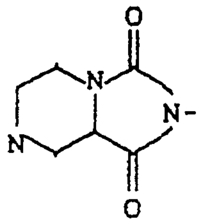 | F | H | H | $-CH_2-C{\equiv}CH$ |
| 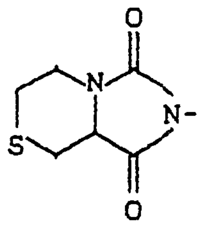 | F | H | H | $-CH_2-C{\equiv}CH$ |
| 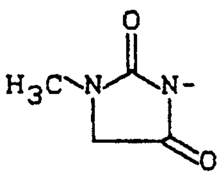 | F | H | H | $-CH_2-C{\equiv}CH$ |
| 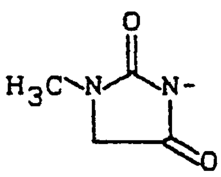 | F | H | H | $-CH(CH_3)_2$ |
| 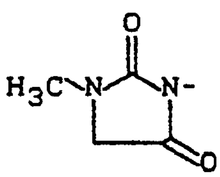 | F | $CH_3$ | H | $-CH_2-C{\equiv}CH$ |

Tabelle 1 - Fortsetzung

| Het | Q | Y | G | Z |
|---|---|---|---|---|
| 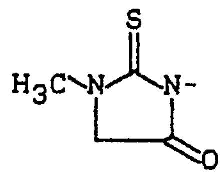 | F | H | H | $-C_2H_5$ |
| 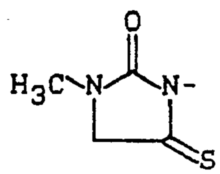 | F | H | $CH_3$ | $-C_3H_7$-n |
| 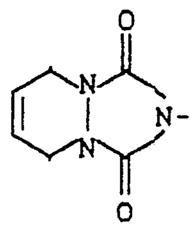 | F | H | H | $-CH_2C{\equiv}CH$ |
| 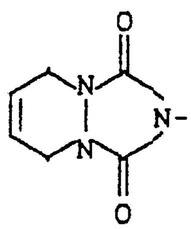 | F | H | H | $-C_2H_5$ |
| 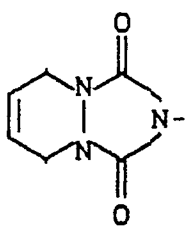 | F | H | H | $-CH_2-CN$ |
| 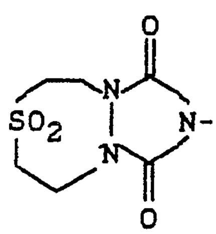 | F | H | H | $-CH_2-C{\equiv}CH$ |

Tabelle 1 - Fortsetzung

| Het | Q | Y | G | Z |
|---|---|---|---|---|
| 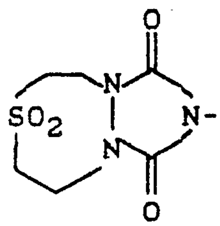 | F | H | H | $-CH_2-CH=CH_2$ |
| 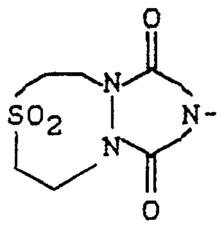 | F | H | H | $-C_2H_5$ |
| 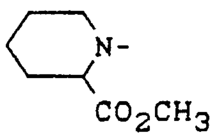 | F | H | H | $-CH_2C\equiv CH$ |
| 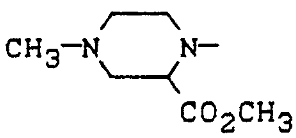 | F | H | H | $-CH_2-CH=CH_2$ |
| 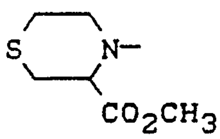 | F | H | H | $-C_2H_5$ |
| 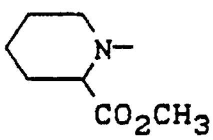 | F | H | H | $-CH_2-C\equiv N$ |
| 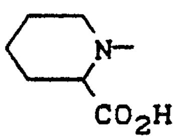 | F | H | H | $-CH_2CF_3$ |

Tabelle 1 - Fortsetzung

| Het | Q | Y | G | Z |
|---|---|---|---|---|
| 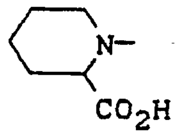 | F | H | H | $-CH_2CH_2OCH_3$ |
| 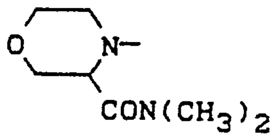 | F | H | H | $-C_4H_9$-n |
| 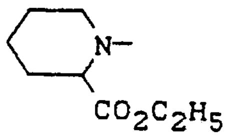 | F | H | H | $-CH_2C{\equiv}C-CH_3$ |
| 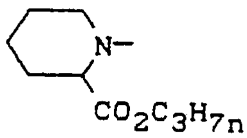 | F | H | H | $-CH_2CO_2C_4H_9$-n |
| 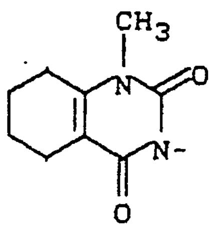 | F | H | H | $-CH_2-C{\equiv}CH$ |
| 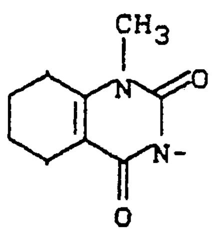 | F | H | H | $-CH(CH_3)_2$ |
| 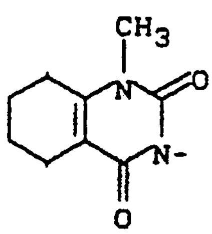 | F | H | H | $-C_3H_7$-n |

Tabelle 1 - Fortsetzung

| Het | Q | Y | G | Z |
|---|---|---|---|---|
| 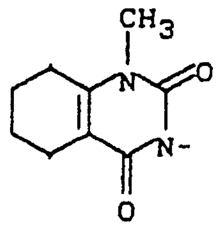 | F | $CH_3$ | H | $-C_4H_9$-n |
| 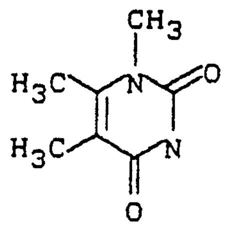 | F | H | H | $-CH_2-C{\equiv}CH$ |
| 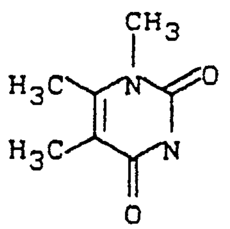 | F | H | H | $-CH(CH_3)_2$ |
| 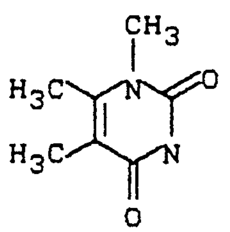 | F | H | H | $-C_3H_7$-n |
| 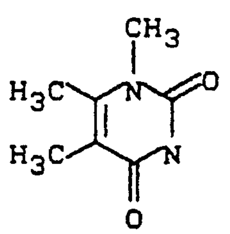 | F | H | $CH_3$ | $-C_4H_9$-n |

Tabelle 1 - Fortsetzung

| Het | Q | Y | G | Z |
|---|---|---|---|---|
| 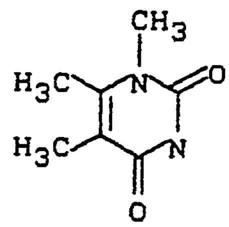 | F | H | H | $-CH_2-C\equiv CH$ |
| 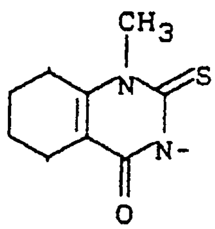 | F | H | H | $-CH_2-C\equiv CH$ |
| 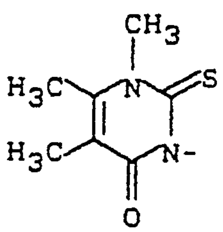 | F | H | H | $-CH_2-C\equiv CH$ |
| 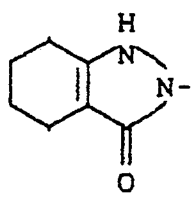 | F | H | H | $-CH_2C\equiv CH$ |
| 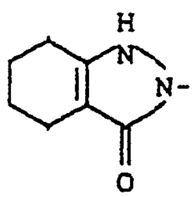 | F | H | H | $-CH_2-CH=CH_2$ |
| 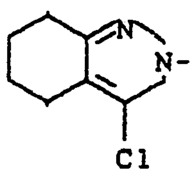 | F | H | H | $-CH_2-C\equiv CH$ |

Tabelle 1 - Fortsetzung

| Het | Q | Y | G | Z |
|---|---|---|---|---|
| 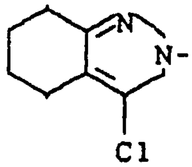 | F | H | H | $-CH_2-CH_2=CH_2$ |
| 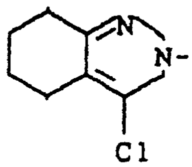 | F | $CH_3$ | H | $-CH(CH_3)_2$ |
| 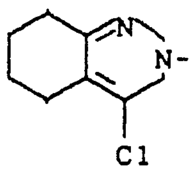 | F | H | $CH_3$ | $-C(=O)-C_2H_5$ |
| 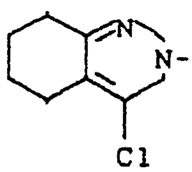 | Cl | H | H | $-C_2H_5$ |
| 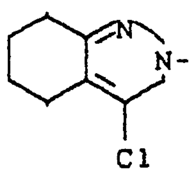 | H | H | H | $-C_4H_9-n$ |
| 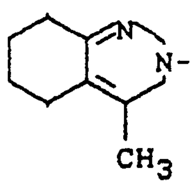 | F | H | H | $-C(=O)-N(CH_3)_2$ |
| 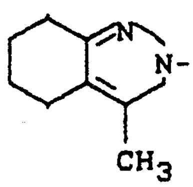 | F | H | H | $-C(=O)-OC_3H_7-i$ |

Tabelle 1 - Fortsetzung

| Het | Q | Y | G | Z |
|---|---|---|---|---|
| 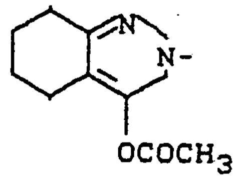 | F | H | H | $-CH_3$ |
| 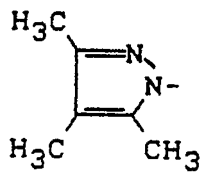 | F | H | H | $-CH_2CF_3$ |
| 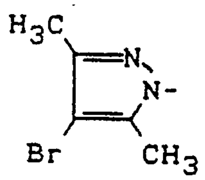 | F | H | H | $-CH_2-C{\equiv}CH$ |
| 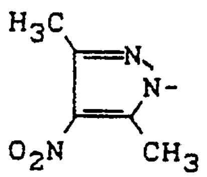 | F | H | H | $-C_4H_9$-n |
| 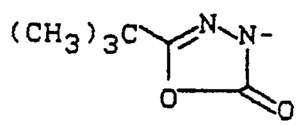 | F | H | H | $CH_2-C{\equiv}CH$ |
| 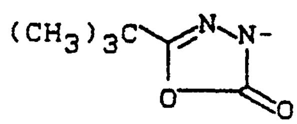 | F | H | H | $CH_2-CH{=}CH_2$ |
| 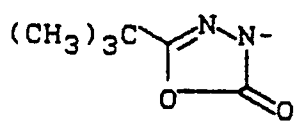 | F | H | H | $C_4H_9$-n |
| 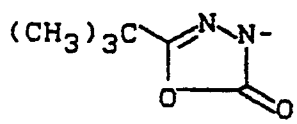 | F | H | H | $C_2H_5$ |

Tabelle 1 - Fortsetzung

| Het | Q | Y | G | Z |
|---|---|---|---|---|
| 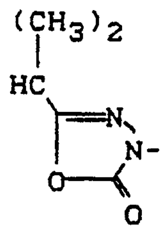 | F | H | H | $CH_2-CN$ |
| 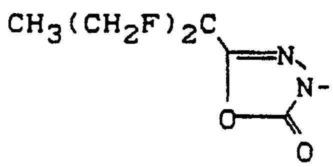 | F | H | H | $CH_2-COOCH_3$ |
| 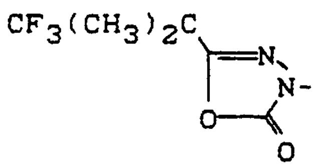 | F | H | H | $CH_2-CF_3$ |
| 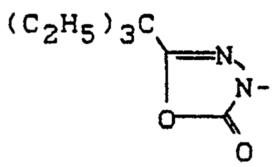 | F | H | H | $CH_3$ |
| 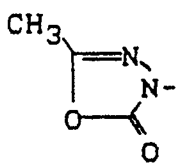 | F | H | H | $C_2H_5$ |
| 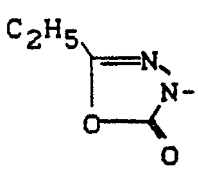 | F | H | H | $CH_2-CH_2OC_2H_5$ |
| 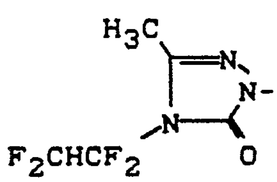 | F | H | H | $-CH_2C{\equiv}CH$ |

Tabelle 1 - Fortsetzung

| Het | Q | Y | G | Z |
|---|---|---|---|---|
| 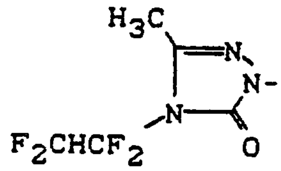 | F | Br | H | $-C_2H_5$ |
| 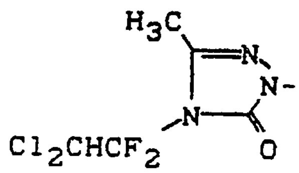 | F | H | H | $-CH_2CH_2OC_3H_{7n}$ |
| 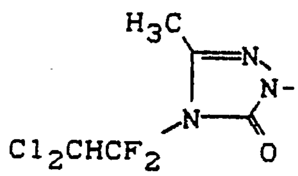 | F | H | H | $-CH_2-C\equiv CH$ |
| 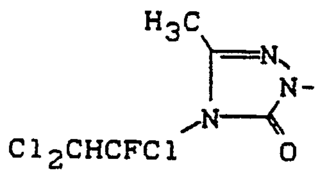 | F | H | H | $CH_2CN$ |
| 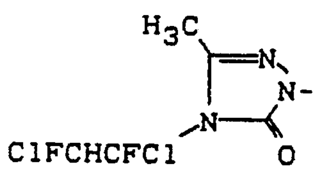 | F | H | H | $CH(CH_3)-C\equiv CH$ |

Verwendet man beispielsweise 3,4,5,6-Tetrahydrophthalsäure-N-(5-fluor-6-indolyl)-imid und Propargylbromid als Ausgangsstoffe, so läßt sich der Reaktionsablauf des erfindungsgemäßen Verfahrens (A) durch das folgende Formelschema darstellen:

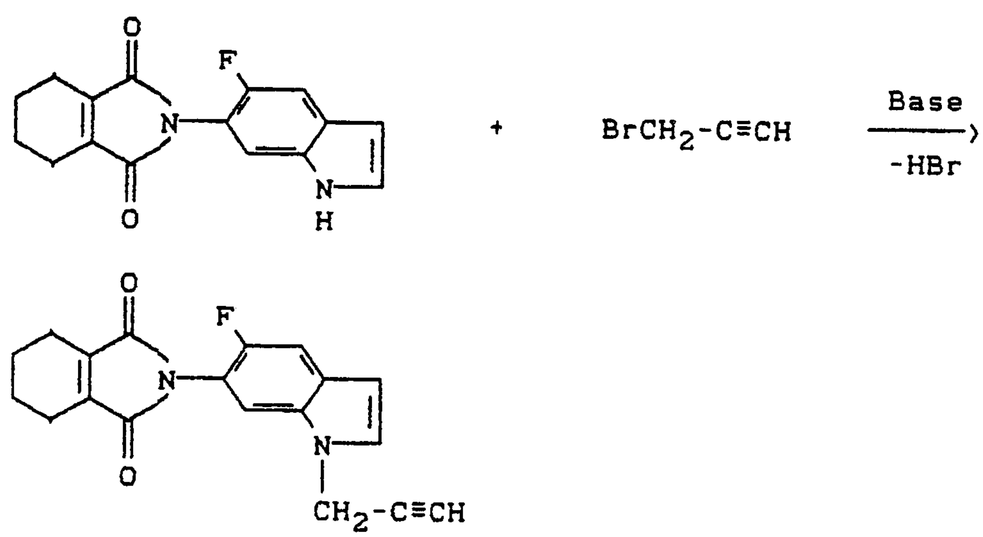

Verwendet man beispielsweise 3,4,5,6-Tetrahydrophthalsäure-N-(5-fluor-6-indolyl)-imid und Propionylchlorid als Ausgangsstoffe, so läßt sich der Reaktionsablauf des erfindungsgemäßen Verfahrens (B) durch das folgende Formelschema darstellen:

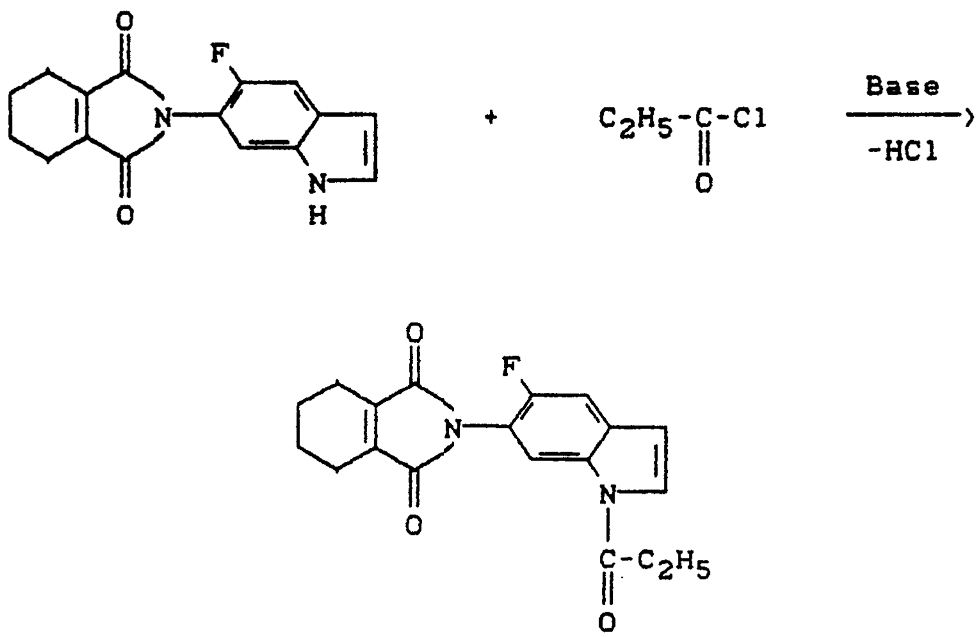


Verwendet man beispielsweise 3,4,5,6-Tetrahydrophthalsäure-N-(5-fluor-6-indolyl)-imid und Methansulfonylchlorid als Ausgangsstoffe, so läßt sich der Reaktionsablauf des erfindungsgemäßen Verfahrens (C) durch das folgende Formelschema darstellen:

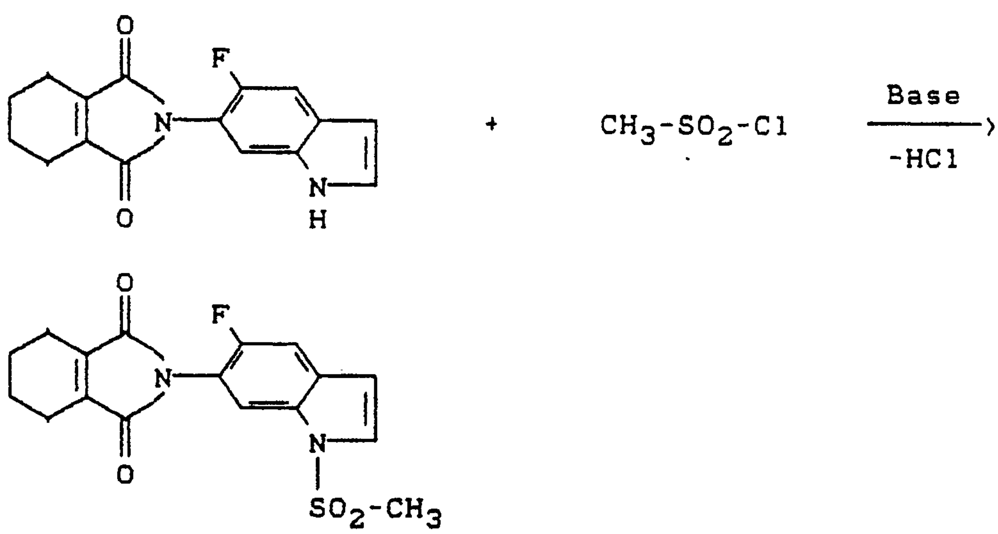


Die Verfahren (D) bis (L) lassen sich beispielhaft durch folgende Reaktionsschemata darstellen:

Verfahren (D):

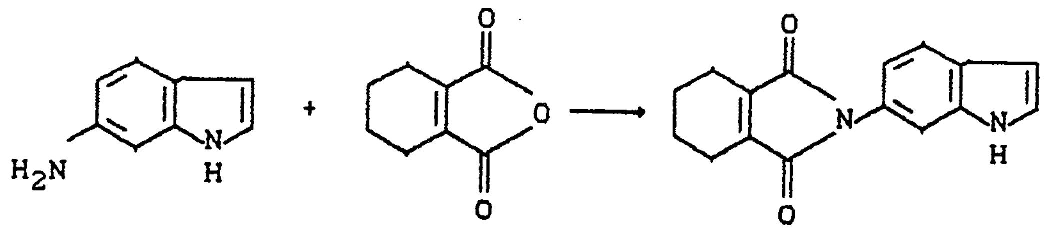

Verfahren (E-α)

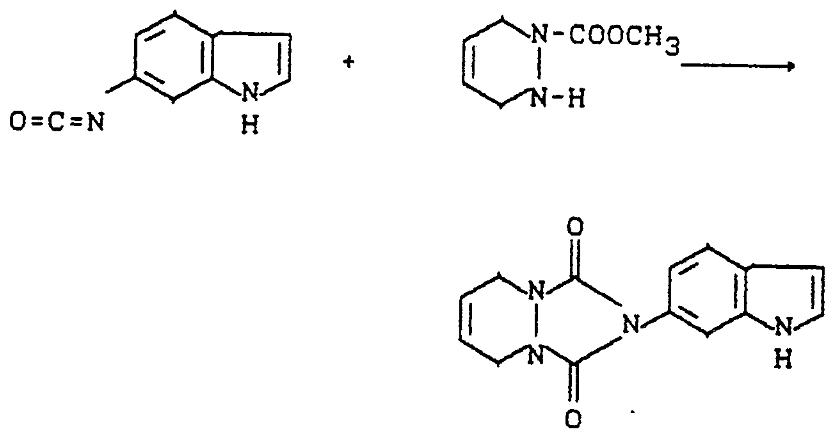

Verfahren (E-β)

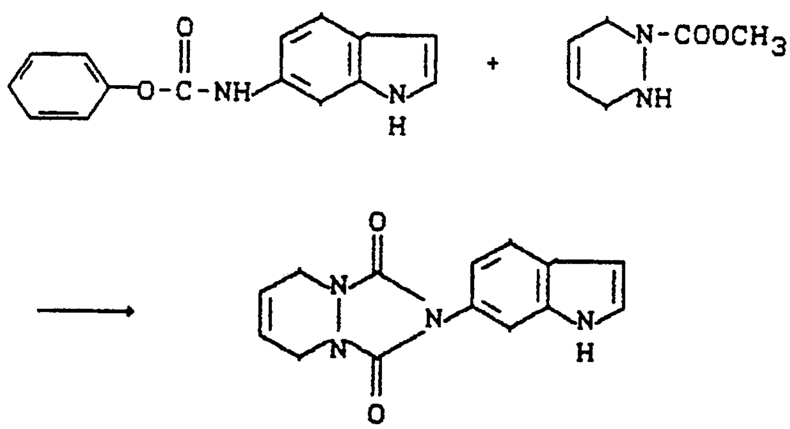

Verfahren (F):

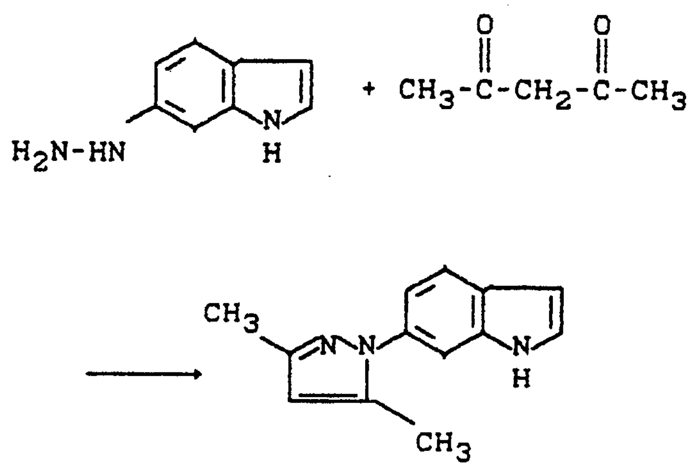

Verfahren (G)

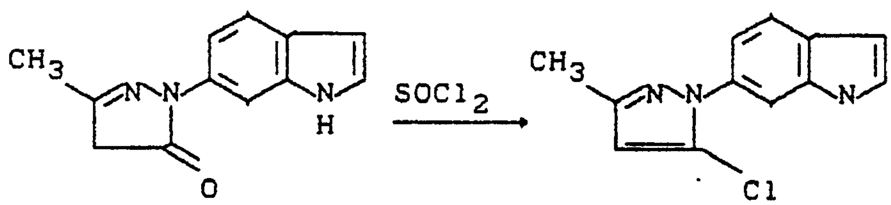


Verfahren (H)

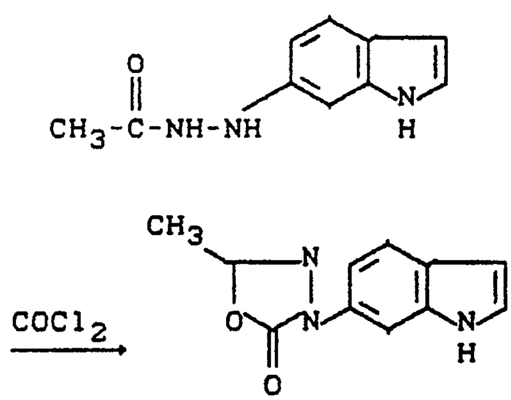


Verfahren (I):

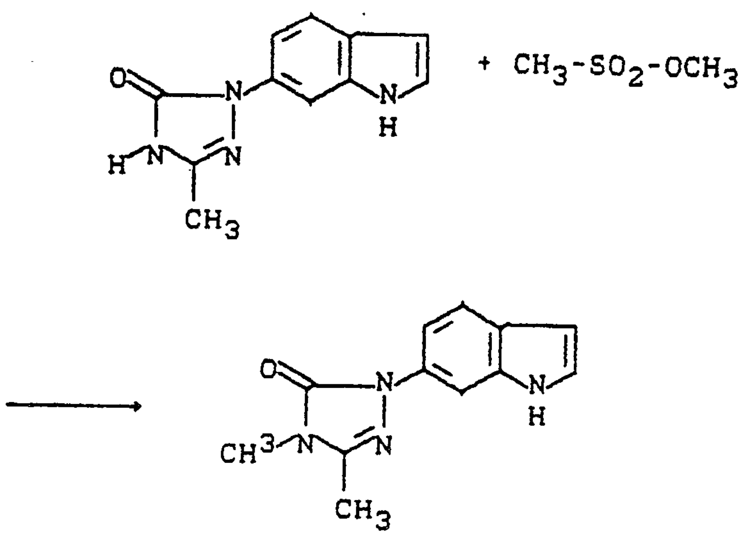


Verfahren (K)

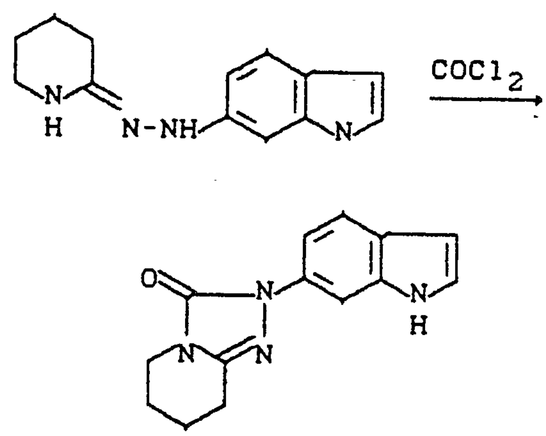


Verfahren (L-α)

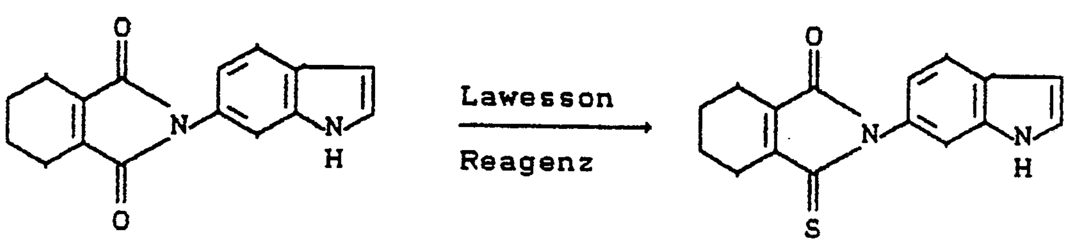


Verfahren (L-β)

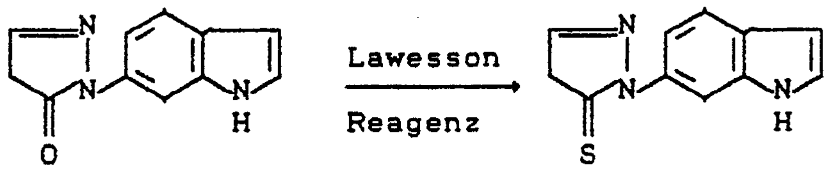


Die zur Durchführung der erfindungsgemäßen Verfahren (A), (B) und (C) als Ausgangsstoffe benötigten Heterocyclen sind durch die Formel (Ib) allgemein definiert. In dieser Formel (Ib) stehen Het, Q, Y und G vorzugsweise für diejenigen Reste, die bereits im Zusammenhang mit der Beschreibung der erfindungsgemäßen Stoffe der Formel (I) als bevorzugt für diese Substituenten genannt wurden. Die Verbindungen der Formel (Ib) sind erfindungsgemäße Verbindungen der Formel (I) mit Z = Wasserstoff.

Die zur Durchführung der erfindungsgemäßen Verfahren (A), (B) und (C) als Ausgangsstoffe benötigten Alkylierungs-, Acylierungs- und Sulfonylierungsmittel der Formeln (II), (IIIa) und (IIIb) sind bekannte Verbindungen der organischen Chemie und/oder lassen sich nach bekannten Verfahren herstellen.

Die zur Durchführung des erfindungsgemäßen Verfahrens (D) als Ausgangsstoffe benötigten 6-Amino-indole sind durch die Formel (IV) allgemein definiert. In dieser Formel (IV) stehen Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

Die 6-Amino-indole der Formel (IV) sind teilweise bekannt (vgl. z.B. EP-OS 0179619, US 3976639, JP 48078164, JP 48057966, Org.Synth. 63, 214-25; J.Org.Chem. 48, 5130-3; Khim. Geterotsikl. Soedin. 493-7; J.Chem.Soc., Perhin Trans. 2, 909-16; Prys. Biochem. 11, 535-46; Khim. Geterotsikl. Soedin. 1381-5; Khim. Geterotsikl. Soedin. 73-8; Khim. Geterotsikl. Soedin., 1428-9) und/oder lassen sich nach bekannten Verfahren in analoger Weise erhalten, indem man beispielsweise 2,4-Dinitrobenzol-Derivate der Formel (XVII)

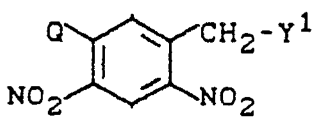

(XVII)

in welcher
Q die oben angegebene Bedeutung hat und
$Y^1$ für Wasserstoff oder Alkyl steht, mit Amidacetalen der Formel (XVIII)

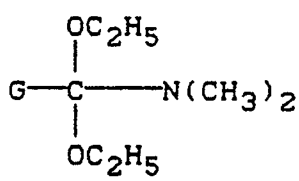

(XVIII)

in welcher
G die oben angegebene Bedeutung hat,
gegebenenfalls in Gegenwart eines Verdünnungsmittels, wie beispielsweise Dimethylformamid bei Temperaturen zwischen 60 und 150 °C zu den Verbindungen der Formel (XIX)

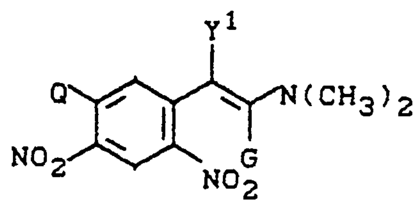

(XIX)

in welcher
Q, G und $Y^1$ die oben angegebene Bedeutung haben,
umsetzt und die so erhaltenen Verbindungen der Formel (XIX), gegebenenfalls nach ihrer Isolierung,

gegebenenfalls in Gegenwart eines Verdünnungsmittels, wie beispielsweise Methanol, gegebenenfalls in Gegenwart eines Inertgases, wie beispielsweise Stickstoff, mit üblichen Reduktionsmitteln, wie beispielsweise Wasserstoff, in Gegenwart eines geeigneten Katalysators, wie beispielsweise Raney-Nickel und einem Druck zwischen 1 und 50 bar, bei Temperaturen zwischen 20 und 100 °C in allgemein üblicher Art und Weise zu den Verbindungen der Formel (IVa),

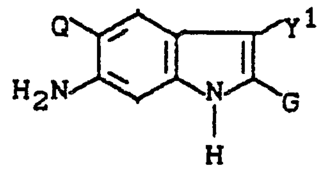

(IVa)

in welcher

Q, G und $Y^1$ die oben angegebene Bedeutung haben,

cyclisiert und anschließend die Verbindungen der Formel (IVa), gegebenenfalls nach ihrer Isolierung, nach allgemein bekannten Methoden, gegebenenfalls in Gegenwart eines Verdünnungsmittels, wie beispielsweise Chloroform, gegebenenfalls zunächst in allgemein bekannter Art und Weise mit üblichen Aminoschutzgruppenreagenzien, wie beispielsweise Acetylchlorid in Gegenwart von Triethylamin an der 6-Aminogruppe schützt (vgl. z.B. Ber. dtsch. chem. Ges. 17 , 609 [1884]), anschließend mit einem Halogenierungsreagenz, wie beispielsweise N-Chlor- oder N-Bromsuccinimid bei Temperaturen zwischen 20 bis 100 °C zu den Verbindungen der Formel (IV) umsetzt und anschließend die Aminoschutzgruppe ebenfalls in allgemein üblicher Art und Weise, wie beispielsweise mit Chlorwasserstoff in Methanol wieder abspaltet.

Die 2,4-Dinitrobenzol-Derivate der Formel (XVII) und die Amidacetale der Formel (XVIII) sind allgemein bekannte Verbindungen der organischen Chemie oder können in Analogie zu bekannten Verfahren hergestellt werden (siehe Beispiel).

Die zur Durchführung des erfindungsgemäßen Verfahrens (D) weiterhin als Ausgangsstoffe benötigten Anhydride sind durch die Formeln (Va)-(Vf) allgemein definiert. In diesen Formeln (Va)-(Vf) stehen $R^1$, $R^2$, X, $X^1$ und $X^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Anhydride der Formeln (Va)-(Vf) sind bekannt oder erhältlich in Analogie zu bekannten Verfahren (vgl. z.B Gazz.Chim.Ital. 57 , 300-311 [1927]; DE-OS 3644222; J. Org.Chem. 51 , 4150-4158 [1986]; Tetrahedron Lett. 25 , 6027-6030 [1984]; J.Org.Chem. 42 , 4162-4164 [1977]; Liebigs Ann. Chem. 1977, 772-790; Tetrahedron 25 , 4099-4108 [1969]; JP 43/9046; J.Chem.Soc., Perhin Trans. 1, 639-48; US 4219652; Helv.Chim.Acta 53, 881-97).

Die zur Durchführung des erfindungsgemäßen Verfahrens (E-α) weiterhin als Ausgangsstoffe benötigten Iso(thio)cyanate sind durch die Formel (V) allgemein definiert. In dieser Formel (V) stehen Q, Y, G und $X^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (VI) sind noch nicht bekannt. Man erhält sie, wenn man 6-Amino-indole der Formel (IV)

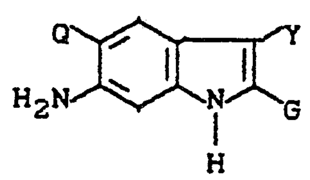

(IV)

in welcher

Q, Y und G die oben angegebene Bedeutung haben,

ggf. an der Indol-NH-Funktion durch eine für diese Reaktion geeignete Schutzgruppe, wie beispielsweise die Acetylgruppe, blockiert und mit Phosgen oder Thiaphosgen, gegebenenfalls in Gegenwart eines Verdünnungsmittels wie beispielsweise Chloroform oder Toluol und gegebenenfalls in Gegenwart eines Reaktionshilfsmittels wie beispielsweise Chlorwasserstoffgas oder Triethylamin bei Temperaturen zwischen -20 °C und +150 °C umsetzt.

Die zur Durchführung des erfindungsgemäßen Verfahrens (E-β) weiterhin als Ausgangsstoffe benötigten Carbamate sind durch die Formel (VII) allgemein definiert. In dieser Formel (VII) stehen Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

Die Carbamate der Formel (VII) sind noch nicht bekannt. Man erhält sie, wenn man 6-Aminoindole der Formel (IV)

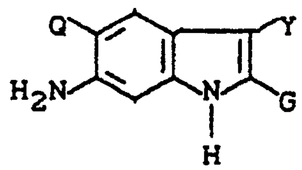

(IV)

in welcher
Q, Y und G die oben angegebene Bedeutung haben,
ggf. an der Indol-NH-Funktion durch eine für diese Reaktion geeignete Schutzgruppe, wie beispielsweise die Acetylgruppe, blockiert und mit Chlorameisensäurephenylester, gegebenenfalls in Gegenwart eines Verdünnungsmittels wie beispielsweise Chloroform und gegebenenfalls in Gegenwart eines Reaktionshilfsmittels wie beispielsweise Triethylamin bei Temperaturen zwischen -20 °C und +150 °C umsetzt.

Die zur Durchführung des erfindungsgemäßen Verfahrens (E-α) und (E-β) als Ausgangsstoffe benötigten Carbonsäureester sind durch die Formel (VIIIa) allgemein definiert. In dieser Formel (VIIIa) steht $R^{13}$ vorzugsweise für einen Rest der Formel

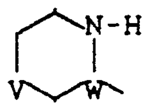 ; 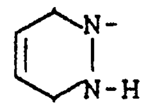 ; 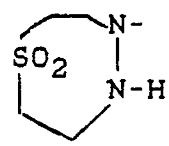 ;

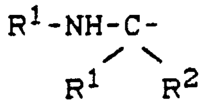 ; 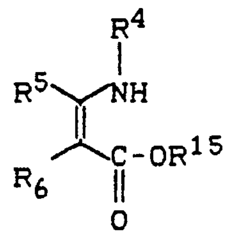 oder

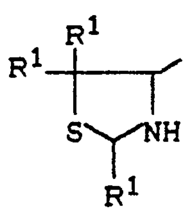

wobei
$R^1$, $R^2$, $R^4$, $R^5$, $R^6$, V und W vorzugsweise für diejenigen Reste stehen, die bereits im Zusammenhang mit der Beschreibung der erfindungsgemäßen Stoffe der Formel (I) als bevorzugt für diese Substituenten genannt wurden.
$R^{14}$ und $R^{15}$ stehen vorzugsweise für geradkettiges oder verzweigtes Alkyl mit 1 bis 4 Kohlenstoffatomen, insbesondere für Methyl oder Ethyl.

Die Carbonsäureester der Formel(VIIIa)sind allgemein bekannte Verbindungen der organischen Chemie oder erhältlich in Analogie zu bekannten Verfahren (vgl. z.B. US 4730006; Pestic. Sci. 16 , 277-286 [1985]; Chem. Pharm. Bull. 32 , 3934-3944 [1984]; Liebigs Ann Chem. 1983 , 1133-1151; Synthesis 1981 , 915-917; Tetrahedron Lett. 22 , 2485-2486 [1981]; Chem. Ber. 111 , 1058-1076 [1978]; Angew. Chem. 89 , 344-345 [1977]; Chem. Ber. 110 , 942-947 [1977]; Chem. Lett. 1976 , 1095-1096; Angew. Chem. 88 , 295-296 [1976]; DE 2058012; Bull.Chem.Soc. Japan 44 , 474-477 [1971]; J.Chem.Soc. Perkin I, 1987 , 877-884; DE 3702943; DE 2331549; J. Heterocycl. Chem. 6 , 181-185, [1969]); J.Med.Pharm.Chem. 2 553 (1960);

J.Am.Chem.Soc. 79 1650 (1957); Tetrahedron 29 3389 (1973).

Die zur Durchführung des erfindungsgemäßen Verfahrens (E-α) und (E-β) als Ausgangsstoffe benötigten Amine sind durch die Formel (VIIIb) allgemein definiert. In dieser Formel (VIIIb) stehen $R^3$ und V für diejenigen Reste, die bereits im Zusammenhang mit der Beschreibung der erfindungsgemäßen Stoffe der Formel (I) als bevorzugt genannt wurden.

Die Amine der Formel (VIIIb) sind allgemein bekannte Verbindungen der organischen Chemie.

Die zur Durchführung des erfindungsgemäßen Verfahrens (F) als Ausgangsstoffe benötigten Hydrazine sind durch die Formel (IX) allgemein definiert. In dieser Formel (IX) stehen Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

Die Hydrazine der Formel (IX) sind noch nicht bekannt und ebenfalls Gegenstand der Erfindung.

Man erhält sie, wenn man 6-Amino-indole der Formel (IV)

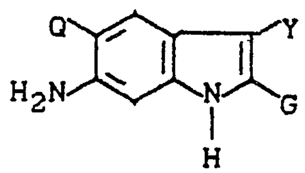

(IV)

in welcher

Q, Y und G die oben angegebene Bedeutung haben,

an der Indol-NH-Funktion durch eine für diese Reaktion geeignete Schutzgruppe, wie beispielsweise Acetyl blockiert, zunächst in üblicher Art und Weise mit Natrium nitrit in Gegenwart einer Säure wie beispielsweise Salzsäure diazotiert und anschließend ebenfalls in allgemein üblicher Art und Weise mit einem Reduktionsmittel wie beispielsweise Zinn(II)chlorid reduziert.

Die zur Durchführung des erfindungsgemäßen Verfahrens (F) weiterhin als Ausgangsstoffe benötigten 1,3-Diketone sind durch die Formel (X) allgemein definiert. In dieser Formel (X) stehen $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{9-1}$ steht vorzugsweise für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen, insbesondere für Wasserstoff, Methyl, Ethyl, n-oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl.

Die zur Durchführung der erfindungsgemäßen Verfahren (G) und (L-β) als Ausgangsstoffe benötigten Pyrazolinyl-indole sind durch die Formel (XI) allgemein definiert. In dieser Formel (XI) stehen $R^7$, $R^8$, Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

Die Pyrazolinyl-indole der Formel (XI) sind neu. Man erhält sie, wenn man β-Ketoester der Formel (XX)

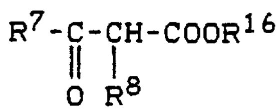

(XX)

in welcher

$R^{16}$ für Alkyl, insbesondere für Methyl oder Ethyl steht und

$R^7$ und $R^8$ die oben angegebene Bedeutung haben, mit Hydrazinen der Formel (IX)

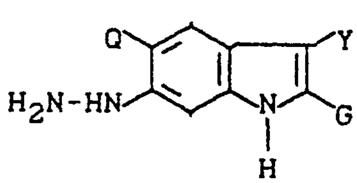

(IX)

in welcher

Q, Y und G die oben angegebene Bedeutung haben,

gegebenenfalls in Gegenwart eines Verdünnungsmittels wie beispielsweise Ethanol und gegebenenfalls in Gegenwart eines Reaktionshilfsmittels wie beispielsweise Schwefelsäure bei Temperaturen zwischen 20 °C und 120 °C umsetzt.

β-Ketoester der Formel (XX) sind allgemein bekannte Verbindungen der organischen Chemie.

Die zur Durchführung des erfindungsgemäßen Verfahrens (H) als Ausgangsstoffe benötigten Hydrazide sind durch die Formel (XII) allgemein definiert. In dieser Formel (XII) stehen $R^{10}$, Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

Die Hydrazide der Formel (XII) sind noch nicht bekannt und Gegenstand der Erfindung.

Man erhält sie, wenn man Hydrazine der Formel (IX)

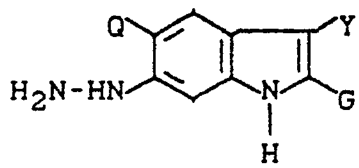

(IX)

in welcher

Q, Y und G die oben angegebene Bedeutung haben, mit Acylierungsmitteln der Formel (XXI)

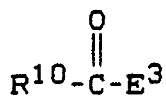

(XXI)

in welcher

$R^{10}$ die oben angegebene Bedeutung hat und

$E^3$ für eine elektronenanziehende Abgangsgruppe, vorzugsweise für Halogen oder für einen Rest

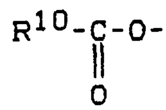

steht, wobei $R^{10}$ die oben angegebene Bedeutung hat und insbesondere für Chlor steht,

gegebenenfalls in Gegenwart eines Verdünnungsmittels wie beispielsweise Dichlormethan und gegebenenfalls in Gegenwart eines Reaktionshilfsmittels wie beispielsweise Triethylamin bei Temperaturen zwischen -20 °C und +60 °C umsetzt.

Acylierungsmittel der Formel (XXI) sind allgemein bekannte Verbindungen der organischen Chemie.

Die zur Durchführung des erfindungsgemäßen Verfahrens (I) als Ausgangsstoffe benötigten Verbindungen der Formel (XIII) sind durch die Formel (XIII) allgemein definiert. In dieser Formel stehen $R^{11}$, Q, Y und G für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (XIII) sind noch nicht bekannt und Gegenstand der Erfindung.

Man erhält sie, wenn man Hydrazine der Formel (IX)

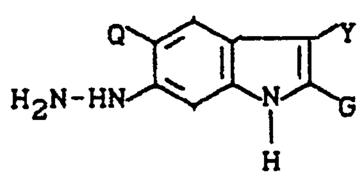

(IX)

in welcher

Q, Y und G die oben angegebene Bedeutung haben, mit Iminocarbonsäureestern der Formel (XXII)

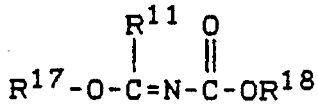

(XXII)

in welcher

$R^{17}$ und $R^{18}$ unabhängig voneinander jeweils für Alkyl stehen und

$R^{11}$ die oben angegebene Bedeutung hat,

gegebenenfalls in Gegenwart eines Verdünnungsmittels, wie beispielsweise Chloroform bei Temperaturen zwischen 10 °C und 80 °C umsetzt.

Die Iminocarbonester der Formel (XXII) sind bekannt oder erhältlich in Analogie zu bekannten oder erhältlich in Analogie zu bekannten Verfahren (vgl. z.B. Chem.Ber. 119 , 2444-2457 [1986]; Bull.Chem.Soc.Jpn. 55 , 3943-3944 [1982]; Chem. Lett. 1982 , 1015-1016; Chem. Lett. 1978 , 1403-1404; J.Am.Chem.Soc. 95 , 3957-3963 [1973]; J.Org. Chem. 36 , 3251-3252 [1971]).

Die zur Durchführung des erfindungsgemäßen Verfahrens (I) weiterhin als Ausgangsstoffe benötigten Alkylierungsmittel sind durch die Formel (XIV) allgemein definiert. In dieser Formel (XIV) steht $R^{12}$ vorzugsweise für diejenige Bedeutung, die bereits im Zusammenhang mit der Beschreibung der erfindungsgemäßen Stoffe der Formel (I) als bevorzugt für diesen Substituenten genannt wurde.

E in Formel (XIV), $E^1$ in Formel (II), $E^2$ in Formel (IIIa) und $E^3$ in Formel (IIIb) stehen vorzugsweise für Halogen, insbesondere für Chlor, Brom oder Iod, oder für jeweils gegebenenfalls substituiertes Alkylsulfonyloxy, Alkoxysulfonyloxy oder Arylsulfanyloxy, wie beispielsweise Methansulfonyloxy, Trifluormethansulfonyloxy, Methoxysulfonyloxy, Ethoxysulfonyloxy oder p-Toluolsulfonyloxy.

Die Alkylierungsmittel der Formel (XIV) sind allgemein bekannte Verbindungen der organischen Chemie.

Die zur Durchführung des erfindungsgemäßen Verfahrens (K) als Ausgangsstoffe benötigten Amidrazone sind durch die Formel (XV) allgemein definiert. In dieser Formel (XV) stehen Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

$R^{11-1}$ und $R^{12-1}$ stehen vorzugsweise gemeinsam für einen zweifach verknüpften Alkandiylrest mit 2 bis 6 Kohlenstoffatomen, insbesondere für einen 1,3-Propandiylrest, einen 1,4-Butandiylrest oder einen 1,5-Pentandiylrest.

Die Amidrazone der Formel (XV) sind noch nicht bekannt. Man erhält sie in Analogie zu bekannten Verfahren (vgl. z.B. US 4080192 oder DE-OS 1957783), wenn man Lactame der Formel (XXIII)

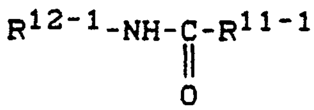

(XXIII)

in welcher

$R^{12-1}$ und $R^{11-1}$ die oben angegebene Bedeutung haben,

zunächst in einer 1. Stufe mit einem Halogenierungsmittel wie beispielsweise Phosphoroxychlorid, Thionylchlorid oder Phosgen gegebenenfalls in Gegenwart eines Verdünnungsmittels wie beispielsweise Ethanol oder Dioxan und gegebenenfalls in Gegenwart eines Reaktionshilfsmittels wie beispielsweise Triethylamin oder Pyridin bei Temperaturen zwischen 0 °C und 50 °C umsetzt und anschließend die so erhältlichen Imidchloride der Formel (XXIV),

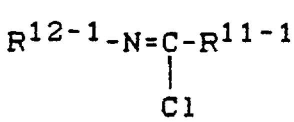

(XXIV)

in welcher

$R^{11-1}$ und $R^{12-1}$ die oben angegebene Bedeutung haben, mit Hydrazinen der Formel (IX)

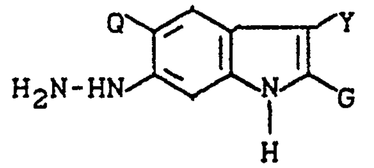

(IX)

in welcher

Q, Y und G die oben angegebene Bedeutung haben,

gegebenenfalls in Gegenwart eines Verdünnungsmittels wie beispielsweise Ethanol und gegebenenfalls in Gegenwart eines Reaktionshilfsmittels wie beispielsweise Triethylamin oder Pyridin bei Temperaturen zwischen 0 °C und 80 °C umsetzt.

Lactame der Formel (XXIV) sind allgemein bekannte Verbindungen der organischen Chemie.

Die zur Durchführung des erfindungsgemäßen Verfahrens (L-α) als Ausgangsstoffe benötigten Heterocyclen sind durch die Formel (Ib-l) allgemein definiert. In dieser Formel (Ib-l) stehen Q, Y und G vorzugsweise für diejenigen Reste, die bereits im Zusammenhang mit der Beschreibung der erfindungsgemäßen Stoffe der Formel (I) als bevorzugt für diese Substituenten genannt wurden.

Het[4] steht vorzugsweise für einen Heterocyclus der Formel

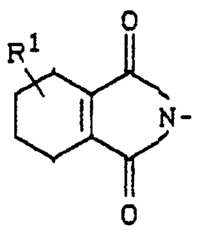 ; 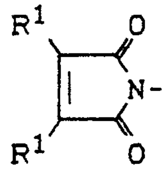 ; 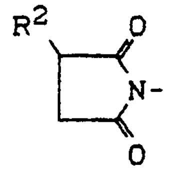 ;

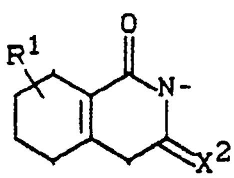 ; 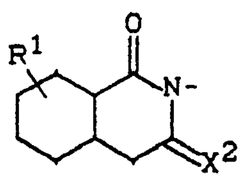 ; 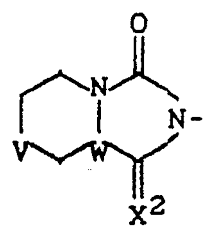 ;

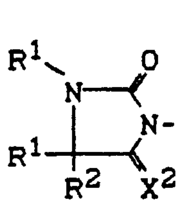 oder 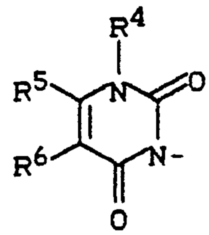

wobei

$X^2$ jeweils für Sauerstoff oder Schwefel steht und

$R^1$, $R^2$, $R^4$, $R^5$, $R^6$, V und W die oben angegebene Bedeutung haben.

Die Heterocyclen der Formel (Ib-l) sind erfindungsgemäße Verbindungen und erhältlich nach Verfahren (D) oder (E).

Das zur Durchführung der erfindungsgemäßen Verfahren (L-α) und (L-β) weiterhin als Ausgangsstoff benötigte "Lawesson Reagenz" ist durch die Formel (XVI) definiert. Das "Lawesson Reagenz" der Formel (XVI) ist bekannt (vgl. z.B. DE-OS 2606083; Bull.Soc.Chim. Belg. 87 , 223-228 [1978]).

Zur Durchführung der erfindungsgemäßen Verfahren (A), (B) und (C) zur Herstellung der neuen N-[Indol-6-yl]-heterocyclen der Formel (I) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether

oder -dimethylether, Ketone, wie Aceton, Butanon, Methylisopropylketon oder Methylisobutylketon, Ester, wie Essigsäureethylester, Säuren, wie Es sigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner in den Varianten (A), (B) oder (C) Verbindungen der Formeln (II), (IIIa) oder (IIIb) in flüssiger Form, so ist es auch möglich, diese in entsprechendem Überschuß gleichzeitig als Verdünnungsmittel einzusetzen.

Als Reaktionshilfsmittel zur Durchführung der erfindungsgemäßen Verfahren (A), (B) oder (C) kommen alle üblicherweise verwendbaren anorganischen und organischen Basen in Frage. Vorzugsweise verwendet man Alkalimetallhydride, -hydroxide, -amide, -carbonate oder -hydrogenoarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (A), (B) oder (C) in einem größeren Bereich variiert werden. Im allgemeinen arbeitet man zwischen -20 °C und +150 °C, vorzugsweise zwischen 0 °C und +100 °C.

Zur Durchführung der erfindungsgemäßen Verfahren (A), (B) oder (C) setzt man pro Mol an Heterocyclen der Formel (Ib) in den Varianten (A), (B) oder (C) im allgemeinen jeweils 1,0 bis 20,0 Mol, vorzugsweise jeweils 1,0 bis 15,0 Mol, an Alkylierungsmittel der Formel (II) oder Acylierungsmittel der Formel (IIIa) oder Sulfonylierungsmittel der Formel (IIIb) und gegebenenfalls 1,0 bis 3,0 Mol, vorzugsweise 1,0 bis 2,0 Mol an Reaktionshilfsmittel, ein. Die Reaktionsdurohführung, Aufarbeitung und Isolierung der Reaktionsprodukte der Formel (I) erfolgt nach üblichen Methoden (vgl. auch die Herstellungsbeisiele).

Als Verdünnungsmittel zur Durchführung des erfindungsgemäßen Verfahrens (D) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Tolual, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (D) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N'-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (D) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (Va)-(Vf) im allgemeinen 1.0 bis 1.5 Mal, vorzugsweise 1.0 bis 1.2 Mol an 6-Amino-indol der Formel (IV) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung der erfindungsgemäßen Verfahren (E-$\alpha$) und (E-$\beta$) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole wie Methanol, Ethanol oder Propanol.

Die erfindungsgemäßen Verfahren (E-$\alpha$) und (E-$\beta$) werden gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (E-$\alpha$) und

(E-$\beta$) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung der erfindungsgemäßen Verfahrens (E-$\alpha$) und (E-$\beta$) setzt man pro Mol an Carbonsäureester der Formel (VIIIa) oder Amin der Formel (VIIIb) oder eines entsprechenden Säureadditionssalzes im allgemeinen entweder 0.5 bis 5.0 Mol, vorzugsweise 0.8 bis 1.5 Mol an Iso(thio)cyanat der Formel (VI) oder 0,5 bis 5,0 Mol, vorzugsweise 0,8 bis 1,5 Mol an Carbamat der Formel (VII) und gegebenenfalls 1.0 bis 10.0 Mol, vorzugsweise 1.0 bis 5.0 Mol an Reaktionshilfsmittel ein. Dabei ist es auch möglich die Isa(thio)-cyanate der Formel (VI) in einer vorgelagerten Reaktion aus 6-Amino-indol der Formel (VI) und Phosgen, Thiophosgen oder Diphosgen ($Cl_3C$-O-CO-Cl) direkt im Reaktiansgefäß herstellen und ohne Isolierung im "Eintopfverfahren" mit den Carbonsäureestern der Formel (VIII) weiter umzusetzen.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahren (F) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid, Alkohole wie Methanol, Ethanol oder Propanol oder Säuren, wie Essigsäure.

Das erfindungsgemäße Verfahren (F) wird gegebenenfalls in Gegenwart eines geeigneten Reaktionshilfsmittels durchgeführt. Als solche kommen insbesondere anorganische Mineralsäuren wie beispielsweise Salzsäure oder Schwefelsäure infrage. Es ist auch möglich, die als Ausgangsstoffe infrage kommenden Hydrazine der Formel (IX) in Form von entsprechenden Säureadditionssalzen, wie beispielsweise Hydrochloriden einzusetzen.

Die Reaktionstemperaturen können bei der Durchführung des erfindungsgemäßen Verfahrens (IX) in einem größeren Bereich variiert werden. Im allgemeinen arbeitet man bei Temperaturen zwischen -20 °C und 180 °C, vorzugsweise bei Temperaturen zwischen 20 °C und 120 °C.

Zur Durchführung des erfindungsgemäßen Verfahrens (F) setzt man pro Mol an Hydrazin der Formel (IX) bzw. an einem entsprechenden Säureadditionssalz im allgemeinen 0.5 bis 10.0 Mol an 1,3-Diketon der Formel (X) oder an einem entsprechenden Derivat und gegebenenfalls 0.01 bis 1.0 Mol an Reaktionshilfsmittel ein. Die Reaktionsdurchführung, Aufarbeitung und Isolierung der Heterocyclen der Formel (Ib-C) erfolgt nach allgemein üblichen Methoden (vgl. auch die Herstellungsbeispiele).

Bei Verwendung von 1,3-Diketonen der Formel (X), bei welchen der Substituent $R^{9-1}$ verschieden von dem Substituenten $R^7$ ist, erhält man in der Regel Isomerengemische aus Verbindungen der Formel (Ib-C1),

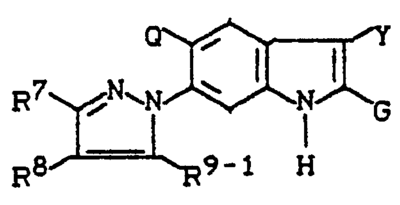

(Ib-C1)

und Verbindungen der Formel (Ib-C2)

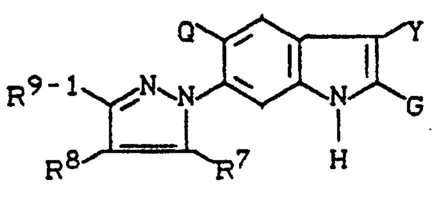

(Ib-C2)

wobei

$R^7$, $R^8$, $R^{9-1}$, Q, Y und G jeweils die oben angegebene Bedeutung haben.

Aus diesen Isomerengemischen lassen sich mit üblichen Trennverfahren (Destillation, Kristallisation, Chromatographie) die gewünschten Reaktionsprodukte der Formel (IIc) isolieren.

Als Halogenierungsmittel zur Durchführung des erfindungsgemäßen Verfahrens (G) kommen ebenfalls übliche Halogenierungsmittel infrage. Mit besonderem Vorzug verwendet man anorganische Säurehalogenide wie beispielsweise Phosphoroxychlorid, Thionylchlorid, Phosgen, Phosphortribromid oder Diphosgen ($Cl_3C$-O-CO-Cl).

Als Verdünnungsmittel zur Durchführung des erfindungsgemäßen Verfahrens (G) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff oder basische Verdünnungsmittel wie beispielsweise Pyridin. Es ist auch möglich, einen entsprechenden Überschuß an Halogenierungsmittel gleichzeitig als Verdünnungsmittel einzusetzen.

Das erfindungsgemäße Verfahren (G) wird vorzugsweise in Gegenwart eines geeigneten Reaktionshilfsmittels durchgeführt. Als solche kommen insbesondere übliche Hilfsnukleophile wie beispielsweise Triphenylphosphin, Dimethylanilin oder Dimethylformamid infrage.

Die Reaktionstemperaturen können bei der Durchführung des erfindungsgemäßen Verfahrens (G) in einem größeren Bereich variiert werden. Im allgemeinen arbeitet man bei Temperaturen zwischen 0 °C und 200 °C, vorzugsweise bei Temperaturen zwischen 30 °C und 150 °C.

Zur Durchführung des erfindungsgemäßen Verfahrens (G) setzt man pro Mol an Pyrazolinyl-indol der Formel (XI) im allgemeinen 1.0 bis 10.0 Mol, vorzugsweise 1.0 bis 5.0 Mol an Halogenierungsmittel und gegebenenfalls 0.01 bis 1.0 Mol, vorzugsweise 0.05 bis 0.1 Mol an Reaktionshilfsmittel ein.
Die Reaktionsdurchführung, Aufarbeitung und Isolierung der Reaktionsprodukte erfolgt nach allgemein üblichen Methoden.

Als Verdünnungsmittel zur Durchführung der erfindungsgemäßen Verfahren (H) und (K) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff.

Die erfindungsgemäßen Verfahren (H) und (K) werden vorzugsweise in Gegenwart eines geeigneten Reaktionshilfsmittels durchgeführt. Als solche kommen alle üblichen Basen in Frage. Mit besonderem Vorzug verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (H) und (K) in einem größeren Bereich variiert werden. Im allgemeinen arbeitet man bei Temperaturen zwischen -20 °C und 180 °C, vorzugsweise bei Temperaturen zwischen 0 °C und 150 °C.

Zur Durchführung des erfindungsgemäßen Verfahrens (K) setzt man pro Mol an Hydrazid der Formel (XV) im allgemeinen 1.0 bis 5.0 Mol, vorzugsweise 1.0 bis 1.5 Mol an Phosgen und gegebenenfalls 1.0 bis 5.0 Mol, vorzugsweise 1.0 bis 3.0 Mol an Reaktionshilfsmittel ein.
Die Reaktionsdurchführung, Aufarbeitung und Isolierung der Reaktionsprodukte erfolgt nach allgemein üblichen Methoden.

Zur Durchführung des erfindungsgemäßen Verfahrens (H) setzt man pro Mol an Hydrazid der Formel (XII) im allgemeinen 1.0 bis 5.0 Mol, vorzugsweise 1.0 bis 1.5 Mol an Phosgen und gegebenenfalls 1.0 bis 5.0 Mol, vorzugsweise 1.0 bis 3.0 Mol an Reaktionshilfsmittel ein.

Die Reaktionsdurchführung, Aufarbeitung und Isolierung der Reaktionsprodukte erfolgt nach allgemein üblichen Methoden.

Zur Durchführung des erfindungsgemäßen Verfahrens (I) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril oder Amide, wie Dimethylformamid, Dimethylacetamid, N-Methylpyrrolidon oder Hexamethylphosphorsäuretriamid. Verwendet man Alkylierungsmittel der Formel (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (I) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat, Kaliumcarbonat oder Natriumhydrogencarbanat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (I) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (I) setzt man pro Mol an Verbindung der Formel (XIII) im allgemeinen 1.0 bis 15.0 Mol, vorzugsweise 1.0 bis 5.0 Mol an Alkylierungsmittel der Formel (XIV) und gegebenenfalls 1.0 bis 3.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung der erfindungsgemäßen Verfahren (L-α) und (L-β) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether.

Die Reaktionstemperaturen können bei der Durchführung der erfindungsgemäßen Verfahren (L-α) und (L-β) in einem größeren Bereich variiert werden. Im allgemeinen arbeitet man bei Temperaturen zwischen 0°C und 180°C, vorzugsweise bei Temperaturen zwischen 20°C und 150°C.

Zur Durchführung der erfindungsgemäßen Verfahren (L-α) und (L-β) setzt man pro Mol an Heterocyclus der Formel (Ib-I) oder (XI) im allgemeinen 0,2 bis 2,0 Mol, vorzugsweise 0,5 bis 1,5 Mol an "Lawesson Reagenz" der Formel ein.

Die Reaktionsdurchführung, Aufarbeitung und Isolierung der Reaktionsprodukte erfolgt in Analogie zu bekannten Verfahren (vgl. z.B. Bull.Soc.Chim.Belg. 87 , 223-228 [1978]).

In der Regel erhält man bei der Durchführung des erfindungsgemäßen Verfahrens (L-α) und (L-β) als Endprodukt Produktgemische, welche sich durch übliche Trennmethoden (Chromatographie, Kristallisation) auftrennen lassen.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrastis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Bekämpfung mono- und dikotyler Unkräuter in mono- und dikotylen Kulturen im Vor- und Nachauflaufverfahren.

Die erfindungsgemäß verwendbaren Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosale, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumaxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze van Eisen, Mangan, Bar, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl )-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; auch Mischungen mit den]-cyclohexancarbonsäure (ALLOXYDIM); 2-Chlor-4-ethyl-2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); exo-1-Methyl-4-(1-methylethyl)-2-(2-methylphenylmethoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N′-(3-trifluormethylphenyl)-harnstoff (FLUOMETURON); [(4-Amino-3,5-dichlor-6-fluor-2-pyridinyl)-oxy]-essigsäure bzw. deren 1-Methylheptylester (FLUROXYPYR); 5-(2-Chlor-4- trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-1H-imidazol-2-yl]-5-ethyl-pyridin-3-carbonsäure (IMAZETHAPYR); 3,5-Diiod-4-hydroxybenzonitril (IOXYNIL); N,N-Dimethyl-N′-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure

(MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chloracetanilid (METALOCHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 2-[4-(6-Chlorchinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)- amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN) sind möglich. Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Verwendung und die Herstellung der erfindungsgemäßen Wirkstoffe geht aus den nachfolgenden Beispielen hervor.

## Herstellungsbeispiele

### Beispiel 1

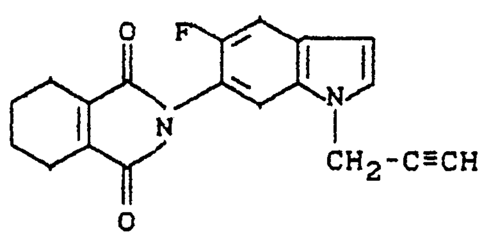

(Verfahrensvariante A)

Zu einem gerührten Gemisch aus 2,72 g (9,57 mmol) 3,4,5,6-Tetrahydrophthalsäure-N-(5-fluor-6-indolyl)-imid, 4,15 g Kaliumcarbonat und 31 ml Aceton gibt man 5,20 g einer 80%igen Lösung von Propargylbromid in Aceton. Man erhitzt bis zur vollständigen Umsetzung unter Rückfluß, läßt abkühlen, versetzt mit Wasser, extrahiert mit Dichlormethan, trocknet den Extrakt über wasserfreiem Natriumsulfat, filtriert, engt ein und reinigt durch Säulenchromatographie an Silicagel (Laufmittel: Dichlormethan).

Man erhält 1,6 g (52% der Theorie) 3,4,5,6-Tetrahydrophthalsäure-N-(2-fluor-1-propargyl-6-indolyl)imid vom Schmelzpunkt 200-201 °C.

In analoger Weise zu Beispiel 1 und unter Berücksichtigung der Angaben in der Beschreibung zu den

erfindungsgemäßen Verfahren, werden die nachfolgend in Tabelle 2 aufgeführten Endprodukte der Formel (I)

Tabelle 2

| Bsp.-Nr. | Het | Q | Y | G | Z | Physikalische Daten |
|---|---|---|---|---|---|---|
| 2 | 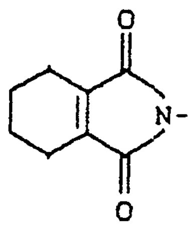 | H | H | H | 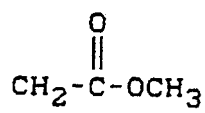 | Fp.: 157-158° C |
| 3 | 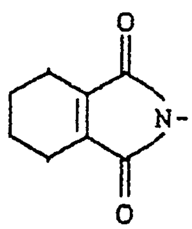 | H | H | H | 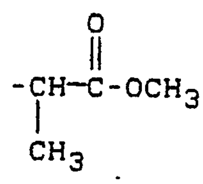 | $^1$H-NMR*) ($CDCl_3$, 200 MHZ, δ = 1,78 (d, 3H), 1,80 (m, 4H), 2,45 (m, 4H), 3,70 (s, 3H), 5,10 (q, 1H) 6,58 (m, 1H) 7,04 (dd, 1H), 7,25 (m, 2H), 7,30 (d, 1H), 7,66 (d, 1H) ppm. |
| 4 | 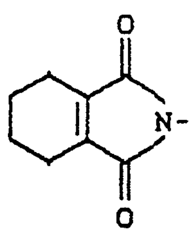 | H | H | H | $-CH_2-C\equiv CH$ | Fp.: 182-185° C |

*)Die $^1$H-NMR-Spektren wurden in Deuterochloroform ($CDCl_3$) mit Tetramethylsilan (TMS) als innerem Standard aufgenommen. Angegebene ist die chemische Verschiebung als δ-Wert in ppm.

Beispiel 5

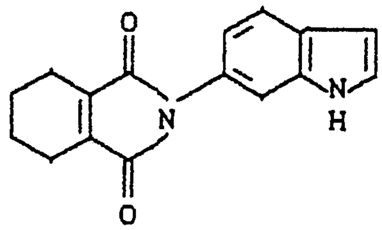

(Verfahrensvariante D)

Ein Gemisch aus 2,64 g (20 mmol) 6-Amino-indol, 2,28 g (15 mmol) 3,4,5,6-Tetrahydrophthalsäureanhydrid und 30 ml Eisessig wird 4 Stunden lang unter Rückfluß erhitzt. Die nach dem Abkühlen ausfallenden Kristalle werden abgesaugt.

Man erhält 2,22 g (56% der Theorie 3,4,5,6-Tetrahydrophthalsäure-N-6-indolyl-imid vom Schmelzpunkt Fp.: 245-248 °C.

Beispiel 6

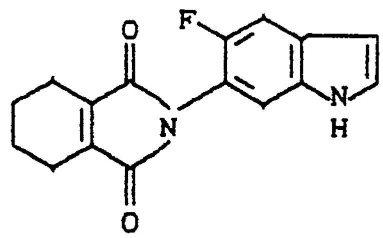

In analoger Weise zu Beispiel 5 und unter Berücksichtigung der Angaben in der Beschreibung zu den erfindungsgemäßen Verfahren wird 3,4,5,6-Tetrahydrophthalsäure-N-(5-fluor-6-indolyl)imid als Beispiel 6 in einer Ausbeute von 99% der Theorie und einem Schmelzpunkt von 208-210 °C erhalten.

Herstellung der Vorprodukte

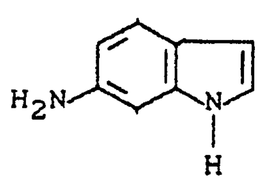

(IV-1)

β-Dimethylamino-2,4-dinitro-styrol wird als Rohprodukt in 400 ml Methanol gelöst und unter Zusatz van Raney-Nickel (20 g) während 3,5 Stunden im Autoklaven bei 50 bar und 50 °C hydriert. Nach dem Abkühlen und Entspannen filtriert man, engt ein und reinigt den Rückstand durch Chromatographie an Kieselgel (Laufmittel: Essigester/Dichlormethan 1:1).

Man erhält 13,50 g (40% der Theorie) 6-Aminoindol. $^{1}$H-NMR (DMSO $d_6$, δ/ppm): δ = 4,65 (s, 2H, $NH_2$), 6,18 (m, 1H), 6,37 (dd, 1H), 6,56 (d, 1H), 6,95 (m, 1H), 7,18 (d, 1H), 10.45 (s, 1H, NH) ppm.

Auf analoge Weise zu Beispiel (IV-1) und unter Berücksichtigung der Angaben in der Beschreibung zu den erfindungsgemäßen Verfahren wird aus β-Dimethylamino-2,4-dinitro-5-fluor-styrol 6-Amino-5-fluor-indol als Beispiel (IV-2) in einer Ausbeute von 43% der Theorie erhalten. $^{1}$H-NMR (DMSO $d_6$, δ/ppm): δ: 4,74 (s, 2H, $NH_2$), 6,20 (m, 1H), 6,75 (d, 1H), 7,07 (m, 1H), 7,10 (d, 1H), 10,60 (s, 1H).

Ein Gemisch aus 47 g (259 mmol) 2,4-Dinitrobenzol, 112 g (761 mmol) Dimethylformamid-diethylacetal

und 200 ml Dimethylformamid wird im 160 °C heißen Ölbad 2-3 Stunden lang erhitzt, wobei man die entstehenden flüchtigen Anteile destillativ abtrennt. Man läßt abkühlen, legt Vakuum an und entfernt bei einer Badtemperatur von bis zu 120 °C auch die schwerer flüchtigen Anteile.

Man erhält in quantitativer Ausbeute einen festen Rückstand van β-Dimethylamino-2,4-dinitrostyrol, der nach GC/MS 93%ig ist und der ohne weitere Reinigung für die nächste Stufe eingesetzt wird. M/e = 237 (81%, $M^{\oplus}$), 220 (30%), 189 (14%), 174 (44%).

Zu einer gerührten Lösung von 25 g (125 mmol) 2,4-Dinitro-5-fluortoluol in 80 ml Toluol läßt man unter Rühren die Hälfte einer Lösung von 27,8 g (188,8 mmol) Dimethylformamid-diethylacetal in 10 ml Toluol tropfen. Man erwärmt langsam auf 60 °C, läßt 2 Stunden bei dieser Temperatur rühren und fügt dann tropfenweise die andere Hälfte der Lösung zu. Nach abermaligem 2stündigem Rühren bei 60 °C läßt man langsam abkühlen, bewahrt über Nacht bei -10 °C auf und saugt die ausgefallenen tiefroten Kristalle ab, die mit kaltem Toluol und n-Hexan gewaschen werden. Aus der Mutterlauge läßt sich weiteres Produkt gewinnen.

Man erhält 23,8 g (75% der Theorie) β-Dimethylamino-2,4-dinitro-5-fluor-styrol mit dem Schmelzpunkt Fp.: 197-198 °C.

92,25 g (0,75 Mol) 3-Fluor-ethylbenzol werden bei 0-5 °C in eine Mischung von 400 g Mischsäure (33 Gew. % Salpetersäure und 67% Schwefelsäure, entsprechend 2,1 Mol Salpetersäure) getropft. Nach beendeter Zugabe wird 30 Minuten bei 0 °C nachgerührt. Durch allmähliches Entfernen der Kühlung steigt die Temperatur der Reaktionsmischung in ca. 2 Stunden auf ca. 15 °C. Bis zum vollständigen Umsatz wird die Reaktionsmischung auf dieser Temperatur gehalten, anschließend auf 500 g Eiswasser gegossen, der Niederschlag abgesaugt und getrocknet.

Nach dem Umkristallisieren aus Tetrachlorkohlenstoff/Hexan erhält man 128 g (80% der Theorie) 5-Fluor-2,4-dinitro-ethylbenzol von Schmelzpunkt 34 °C.

Beispiel A

Post-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % - keine Wirkung (wie unbehandelte Kontrolle)

100 % = totale Vernichtung

Eine sehr gute herbizide Wirksamkeit bei sehr guter Nutzpflanzenselektivität zeigen in diesem Test z.B. die Verbindungen gemäß der Herstellungsbeispiele: (1), (3) und (4).

Beispiel B

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant.

Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine sehr gute herbizide Wirksamkeit und eine sehr gute Nutzpflanzenselektivität zeigen in diesem Test z.B. die Verbindungen gemäß der Herstellungsbeispiele: (1), (4) und (6).

## Ansprüche

1. N-[Indol-6-yl]-heterocyclen der Formel (I),

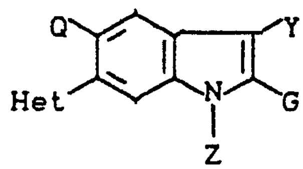

(I)

in welcher

Het für einen Heterocyclus der Formel

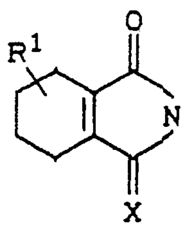 ;

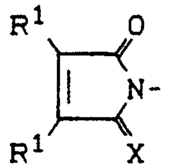 ;

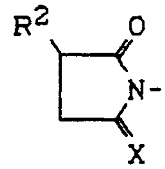 ;

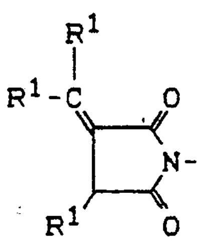 ;

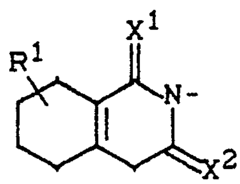 ;

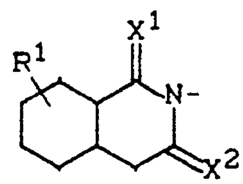 ;

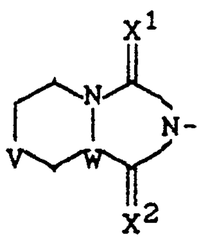 ;

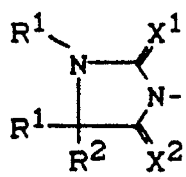 ;

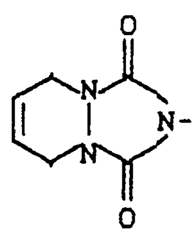 ;

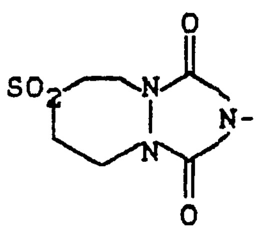 ;

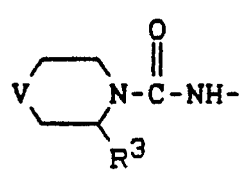 ;

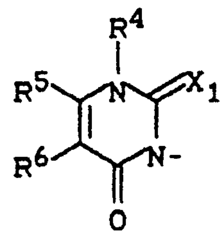 ;

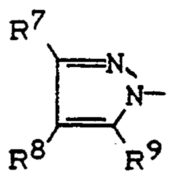 ; 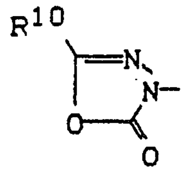 ; 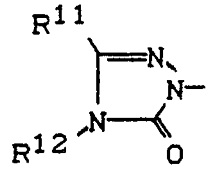 ;

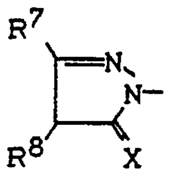 ; 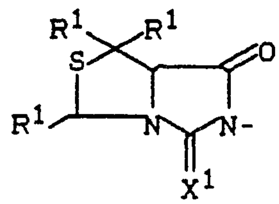 steht,

Q für Wasserstoff oder Halogen steht,
Y für Wasserstoff, Alkyl oder Halogen steht,
G für Wasserstoff oder Alkyl steht,
Z für Wasserstoff, unsubstituiertes oder substituiertes Alkyl, unsubstituiertes oder substituiertes Alkenyl, unsubstituiertes oder substituiertes Alkinyl, unsubstituiertes oder substituiertes Cycloalkyl, unsubstituiertes oder substituiertes Aralkyl, für Halogenalkyl, Alkoxycarbonyl oder (Di)alkylaminocarbonyl, Alkylcarbonyl oder Alkylsulfonyl steht,
$R^1$ für Wasserstoff oder Alkyl mit 1 bis 8 Kohlenstoffatomen steht,
$R^2$ für Wasserstoff, Alkyl mit 1 bis 8 Kohlenstoffatomen oder Halogen steht,
$R^3$ für Cyano oder für den Rest
$-COOR^1$ oder $-CONR^1R^1$
steht,
$R^4$, $R^5$ und $R^6$ entweder unabhängig voneinander jeweils für Wasserstoff oder Alkyl mit 1 bis 8 Kohlenstoffatomen stehen oder jeweils $R^4$ und $R^5$ gemeinsam oder $R^5$ und $R^6$ gemeinsam für eine Alkylkette mit 4 bis 6 Kohlenstoffatomen stehen,
$R^7$ für Wasserstoff, Alkyl mit 1 bis 8 Kohlenstoffatamen oder Halogenalkyl mit 1 bis 8 Kohlenstoffatomen und 1 bis 17 gleichen oder verschiedenen Halogenatomen steht,
$R^8$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^9$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halogenatomen oder Halogen steht
oder
$R^7$ und $R^8$ gemeinsam oder $R^8$ und $R^9$ gemeinsam für eine Alkylkette mit 4 bis 6 Kohlenstoffatomen stehen,
$R^{10}$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halogenatomen oder unsubstituiertes oder substituiertes Cycloalkyl mit 3 bis 7 Kohlenstoffatomen steht,
$R^{11}$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^{12}$ für Alkyl mit 1 bis 8 Kohlenstoffatomen oder Halogenalkyl mit 1 bis 8 Kohlenstoffatomen und 1 bis 17 gleichen oder verschiedenen Halogenatomen steht,
oder
$R^{11}$ und $R^{12}$ gemeinsam für eine Alkylkette mit 4 bis 6 Kohlenstoffatomen stehen,
V für Sauerstoff, Schwefel oder für eine Gruppe $-CH_2-$; $-SO_2-$ oder $-NCH_3$ steht,
W für Stickstoff oder die -CH-Gruppe steht,
X für Sauerstoff, Schwefel oder die $-CH_2$-Gruppe steht,
$X^1$ für Sauerstoff oder Schwefel steht und
$X^2$ für Sauerstoff oder Schwefel steht,
ausgenommen die Verbindungen N-[1-Methyl-indol-6-yl]-phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro-1H-isoindol-1,3(2H)-dion; 2-[5-Fluor-1-(1-methylethyl)-1H-indol-6-yl]-4,5,6,7-tetrahydro-1H-isoindol-1,3(2H)-dion, 2-[5-Fluor-1-(1-methylpropyl)-1H-indol-6-yl]-4,5,6,7-tetrahydro-1H-isoindol-1,3(2H)-dion und 2-(5-Fluor-1H-indol-6-yl)-4,5,6,7-tetrahydro-1H-isoindol-1,3(2H)-dion.

2. N-[Indol-6-yl]-heterocyclen der Formel (I) gemäß Anspruch 1, in welcher

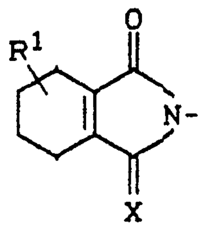 ; 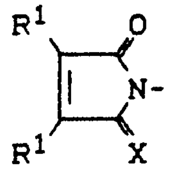 ; 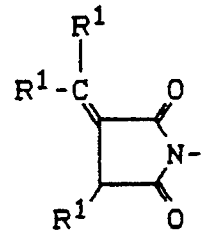

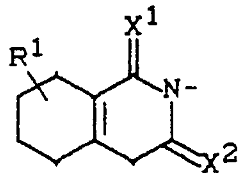 ; 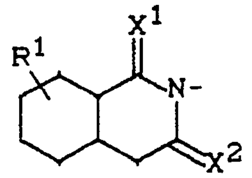 ; 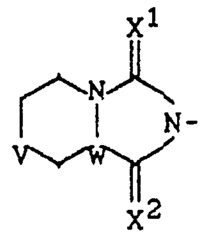

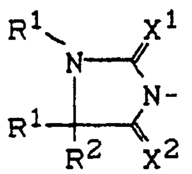 ; 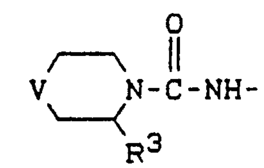 ; . 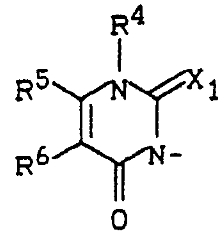

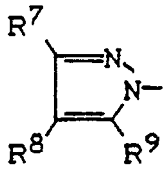 ; 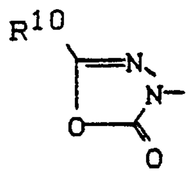 ; 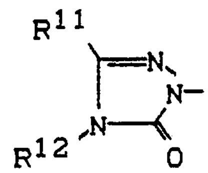 ;

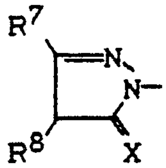 oder 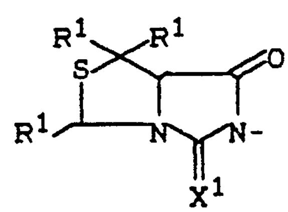 steht,

Q für Wasserstoff, Fluor, Chlor oder Brom steht,

Y für Wasserstoff, Alkyl mit 1 bis 4 Kohlenstoffatomen oder Fluor, Chlor oder Brom steht,

G für Wasserstoff oder Alkyl mit 1 bis 4 Kohlenstoffatomen steht,

Z für Wasserstoff, unsubstituiertes oder substituiertes Alkyl mit 1 bis 8 Kohlenstoffatomen, für unsubstituiertes oder substituiertes Alkenyl mit 2 bis 8 Kohlenstoffatomen, für unsubstituiertes oder substituiertes Alkinyl mit 3 bis 8 Kohlenstoffatomen, wobei als Substituenten jeweils Methoxy, Ethoxy, n- oder i-Propoxy, n-, i-, s- oder t-Butoxy, Cyano, Alkoxycarbonyl mit 1 bis 4 Kohlenstoffatomen oder Cyclohexyl mit 3 bis 7 Kohlenstoffatomen in Frage kommen; für unsubstituiertes oder einfach oder mehrfach, gleich oder verschieden substituiertes Cycloalkyl mit 3 bis 6 Kohlenstoffatomen, wobei als Substituenten Fluor, Chlor, Methyl, Ethyl, n- oder i-Propyl genannt seien; unsubstituiertes oder einfach oder mehrfach, gleich oder verschieden substituiertes Phenylalkyl mit 1 bis 4 Kohlenstoffatomen im Alkylteil, wobei als Substituenten in Frage kommen:

Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 oder 2 Kohlenstoffatomen und 1 bis 5

gleichen oder verschiedenen Halogenatomen;
Z steht weiterhin für Halogenalkyl mit 1 bis 8 Kohlenstoffatomen und 1 bis 17 gleichen oder verschiedenen Halogenatomen, Alkoxycarbonyl mit 1 bis 8 Kohlenstoffatomen im Alkoxyteil, (Di)alkylaminocarbonyl mit 1 bis 8 Kohlenstoffatomen in den einzelnen Alkylteilen, Acyl mit 1 bis 4 Kohlenstoffatamen oder Alkylsulfonyl mit 1 bis 4 Kohlenstoffatomen,
$R^1$ für Wasserstoff oder Alkyl mit 1 bis 4 Kohlenstoffatomen steht,
$R^2$ für Wasserstoff, Alkyl mit 1 bis 4 Kohlenstoffatomen, Fluor oder Chlor steht,
$R^3$ für Cyano oder für den Rest
$-COOR^1$ oder $-CONR^1R^1$
steht,
$R^4$, $R^5$ und $R^6$ entweder unabhängig voneinander jeweils für Wasserstoff oder Alkyl mit 1 bis 4 Kohlenstoffatomen stehen oder jeweils $R^4$ und $R^5$ gemeinsam oder $R^5$ und $R^6$ gemeinsam für eine Alkylkette mit 4 oder 5 Kohlenstoffatomen stehen,
$R^7$ für Wasserstoff, Alkyl mit 1 bis 4 Kohlenstoffatomen oder Halogenalkyl mit 1 bis 4 Kohlenstoffatomen und 1 bis 9 gleichen oder verschiedenen Halogenatomen steht,
$R^8$ für Wasserstoff, Alkyl mit 1 bis 4 Kohlenstoffatomen oder Halogenalkyl mit 1 bis 4 Kohlenstoffatomen und 1 bis 9 gleichen oder verschiedenen Halogenatomen steht,
$R^9$ für Wasserstoff, Fluor, Chlor, Brom, Alkyl mit 1 bis 4 Kohlenstoffatomen oder Halogenalkyl mit 1 bis 4 Kohlenstoffatomen und 1 bis 9 gleichen oder verschiedenen Halogenatomen steht oder
$R^7$ und $R^8$ gemeinsam oder $R^8$ und $R^9$ gemeinsam für eine Alkylkette mit 4 oder 5 Kohlenstoffatomen stehen,
$R^{10}$ für Wasserstoff, Alkyl mit 1 bis 4 Kohlenstoffatomen, Halogenalkyl mit 1 bis 4 Kohlenstoffatomen und 1 bis 9 gleichen oder verschiedenen Halogenatomen oder unsubstituiertes oder einfach bis dreifach, gleich oder verschieden durch Methyl, Ethyl, n-oder i-Propyl, Fluor oder Chlor substituiertes Cycloalkyl mit 3 bis 5 Kohlenstoffatomen steht,
$R^{11}$ für Wasserstoff, Alkyl mit 1 bis 4 Kohlenstoffatomen oder Halogenalkyl mit 1 bis 4 Kohlenstoffatomen und 1 bis 9 gleichen oder verschiedenen Halogenatomen steht,
$R^{12}$ für Alkyl mit 1 bis 4 Kohlenstoffatomen oder Halogenalkyl mit 1 bis 4 Kohlenstoffatomen und 1 bis 9 gleichen oder verschiedenen Halogenatomen steht,
oder
$R^{11}$ und $R^{12}$ gemeinsam für eine Alkylkette mit 4 oder 5 Kohlenstoffatomen steht,
V für Sauerstoff, Schwefel oder für eine Gruppe $-CH_2-$; $SO_2$ oder $-NCH_3$ steht,
W für Stickstoff oder die -CH-Gruppe steht,
X für Sauerstoff, Schwefel oder die $-CH_2$-Gruppe steht,
$X^1$ für Sauerstoff oder Schwefel steht und
$X^2$ für Sauerstoff oder Schwefel steht,
ausgenommen die Verbindungen N-[1-Methyl-indol-6-yl]-phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro- 1H-isoindol-1,3(2H)-dion; 2-[5-Fluor-1-(1-methylethyl)-1H-indol-6-yl]-4,5,6,7-tetrahydro-1H-isoindol-1,3(2H)-dion, 2-[5-Fluor-1-(1-methylpropyl)-1H-indol-6-yl]-4,5,6,7-tetrahydro-1H-isoindol-1,3(2H)-dion und 2-(5-Fluor-1H-indol-6-yl)-4,5,6,7-tetrahydro-1H-isoindol-1,3(2H)-dion.

3. Verfahren zur Herstellung von N-[Indol-6-yl]-heterocyclen der Formel (I),

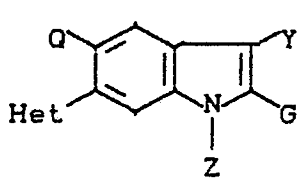

(I)

in welcher
Het für einen Heterocyclus der Formel

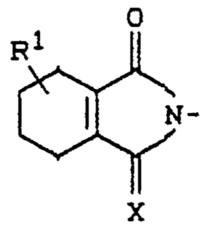 ; 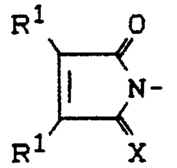 ; 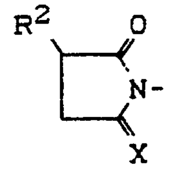 ;

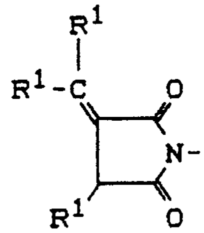 ; 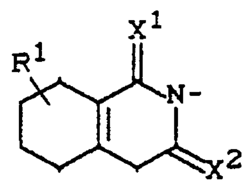 ; 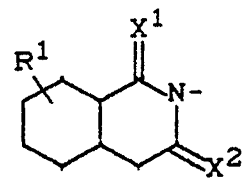 ;

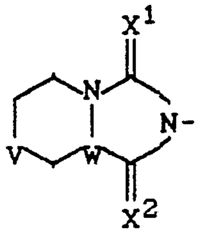 ; 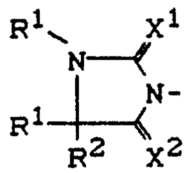 ; 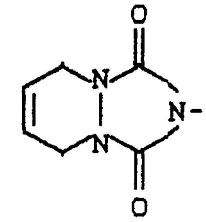 ;

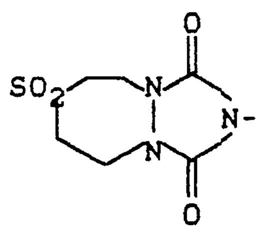 ; 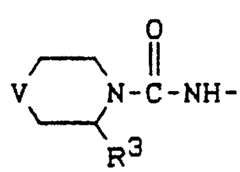 ; 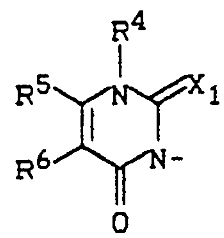 ;

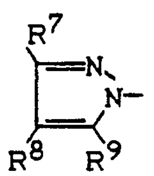 ; 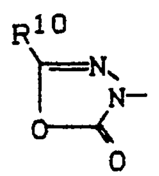 ; 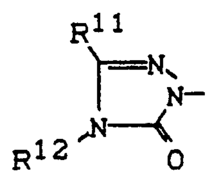 ;

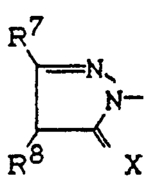 ; 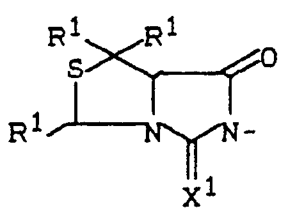 steht,

Q für Wasserstoff oder Halogen steht,

Y für Wasserstoff, Alkyl oder Halogen steht,

G für Wasserstoff oder Alkyl steht,

Z für Wasserstoff, unsubstituiertes oder substituiertes Alkyl, unsubstituiertes oder substituiertes Alkenyl, unsubstituiertes oder substituiertes Alkinyl, unsubstituiertes oder substituiertes Cycloalkyl, unsubstituiertes oder substituiertes Aralkyl, für Halogenalkyl, Alkoxycarbonyl oder (Di)alkylaminocarbonyl, Alkylcarbonyl oder Alkylsulfonyl steht,

$R^1$ für Wasserstoff oder Alkyl mit 1 bis 8 Kohlenstoffatomen steht,

$R^2$ für Wasserstoff, Alkyl mit 1 bis 8 Kohlenstoffatomen oder Halogen steht,

$R^3$ für Cyano oder für den Rest
-$COOR^1$ oder -$CONR^1R^1$
steht,
$R^4$, $R^5$ und $R^6$ entweder unabhängig voneinander jeweils für Wasserstoff oder Alkyl mit 1 bis 8 Kohlenstoffatomen stehen oder jeweils $R^4$ und $R^5$ gemeinsam oder $R^5$ und $R^6$ gemeinsam für eine Alkylkette mit 4 bis 6 Kohlenstoffatomen stehen,
$R^7$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^8$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,
$R^9$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halagenatomen oder Halogen steht
oder
$R^7$ und $R^8$ gemeinsam oder $R^8$ und $R^9$ gemeinsam für eine Alkylkette mit 4 bis 6 Kohlenstoffatomen stehen,
$R^{10}$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halogenatomen oder unsubstituiertes oder substituiertes Cycloalkyl mit 3 bis 7 Kohlenstoffatomen steht,
$R^{11}$ für Wasserstoff, Alkyl mit 1 bis 8 Kohlenstoffatomen oder Halagenalkyl mit 1 bis 8 Kohlenstoffatomen und 1 bis 17 gleichen oder verschiedenen Halogenatomen steht,
$R^{12}$ für Alkyl mit 1 bis 8 Kohlenstoffatomen oder Halogenalkyl mit 1 bis 8 Kohlenstoffatomen und 1 bis 17 gleichen oder verschiedenen Halogenatomen steht,
oder
$R^{11}$ und $R^{12}$ gemeinsam für eine Alkylkette mit 4 bis 6 Kohlenstoffatomen stehen,
V für Sauerstoff, Schwefel oder für eine Gruppe -$CH_2$-; -$SO_2$- oder -$NCH_3$ steht,
W für Stickstoff oder die -CH-Gruppe steht,
X für Sauerstoff, Schwefel oder die -$CH_2$-Gruppe steht,
$X^1$ für Sauerstoff oder Schwefel steht und
$X^2$ für Sauerstoff oder Schwefel steht,
ausgenommen die Verbindungen N-[1-Methyl-indol-6-yl]-phthalimid; 2-(1-Ethyl-5-fluor-1H-indol-6-yl)-4,5,6,7-tetrahydro-1H-isoindol-1,3(2H)-dion; 2-(5-fluor-1-propyl-1H-indol-6-yl)-4,5,6,7-tetrahydro-1H-isoindol-1,3(2H)-dion; 2-[5-Fluor-1-(1-methylethyl)-1H-indol-6-yl]-4,5,6,7-tetrahydro-1H-isoindol-1,3(2H)-dion, 2-[5-Fluor-1-(1-methylpropyl)-1H-indol-6-yl]-4,5,6,7-tetrahydro-1H-isoindol-1,3(2H)-dion und 2-(5-Fluor-1H-indol-6-yl)-4,5,6,7-tetrahydro-1H-isoindol-1,3(2H)-dion. dadurch gekennzeichnet, daß man N-[Indol-6-yl]-heterocyclen der allgemeinen Formel (Ia)

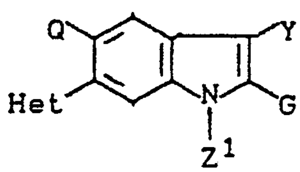

(Ia)

in welcher
$Z^1$ für die oben angegebenen Bedeutungen mit Ausnahme von Wasserstoff steht, Het, Q, G und Y die oben angegebenen Bedeutungen haben, ausgenommen die bereits oben genannten Verbindungen,
erhält, wenn man Heterocyclen der allgemeinen Formel (Ib)

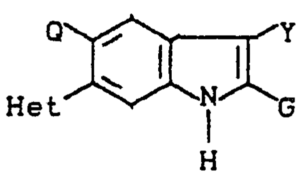

(Ib)

in welcher
Het, Q, Y und G die oben angegebene Bedeutung haben,

A) mit Alkylierungsmitteln der Formel (II)

$Z^1$-$E^1$ (II)

in welcher

$Z^1$ für unsubstituiertes oder substituiertes Alkyl, unsubstituiertes oder substituiertes Alkenyl, unsubstituiertes oder substituiertes Alkinyl, unsubstituiertes oder substituiertes Cycloalkyl, unsubstituiertes oder substituiertes Aralkyl oder Halogenalkyl steht und

$E^1$ für eine elektronenanziehende Abgangsgruppe steht,

oder

B) mit Acylierungsmitteln der Formel (IIIa)

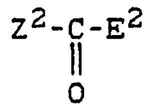

(IIIa)

in welcher

$Z^2$ für Alkoxy, (Di)alkylamino oder Alkyl steht und

$E^2$ für eine elektronenanziehende Abgangsgruppe steht,

oder

C) mit Sulfonylierungsmitteln der Formel (IIIb)

$Z^3$-$SO_2$-$E^3$ (IIIb)

in welcher

$Z^3$ für Alkyl steht und

$E^3$ für eine elektronenanziehende Abgangsgruppe steht,

jeweils gegebenenfalls in Gegenwart eines Verdünnungsmittels und gegebenenfalls in Gegenwart eines Reaktionshilfsmittels umsetzt, oder daß man

N-[Indol-6-yl]-heterocyclen der Formel (Ib), in denen Z für Wasserstoff steht, nach einem der im Folgenden beschriebenen Verfahren erhält:

D) Man erhält N-[Indol-6-yl]-heterocyclen der allgemeinen Formel (Ib-A)

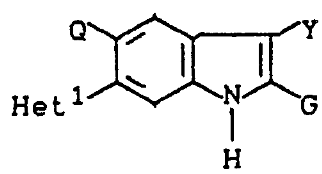

(Ib-A)

in welcher

$Het^1$ für einen Heterocyclus der Formel

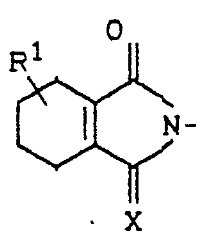 ; 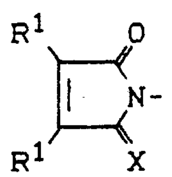 ; 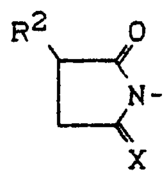 ;

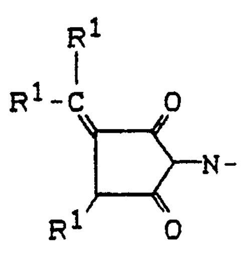 ; 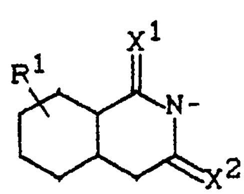 oder 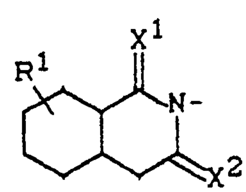

steht und
Q, Y, G, $R^1$, $R^2$, X, $X^1$ und $X^2$ die oben angegebene Bedeutung haben,
wenn man 6-Amino-indole der Formel (IV)

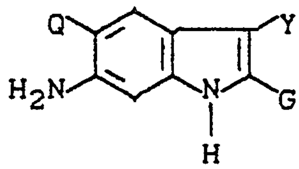

(IV)

in welcher
Q, Y und G die oben angegebene Bedeutung haben, mit Anhydriden der Formel (Va) - (Vf)

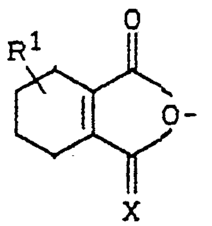 ; 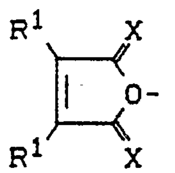 ; 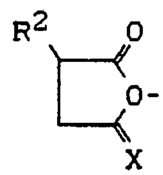 ;

(Va) (Vb) (Vc)

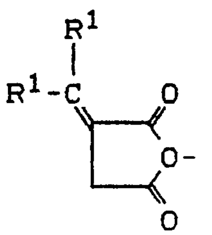 ; 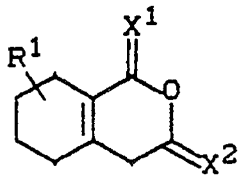 ; 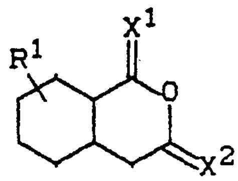

(Vd) (Ve) (Vf)

in welcher
$R^1$, $R^2$, X, $X^1$ und $X^2$ die oben angegebene Bedeutung haben,
gegebenenfalls in Gegenwart eines Verdünnungsmittels und gegebenenfalls in Gegenwart eines Reaktionshilfsmittels umsetzt;
oder
E) man erhält Heterocyclen der allgemeinen Formel (Ib-B)

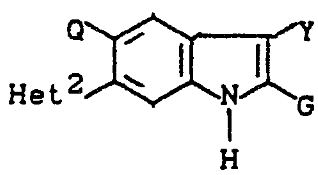

(Ib-B)

in welcher
$Het^2$ für einen Heterocyclus der Formel

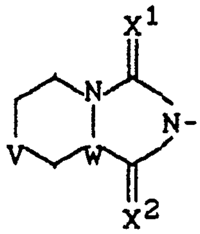 ; 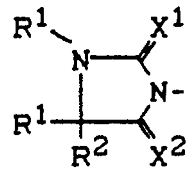 ; 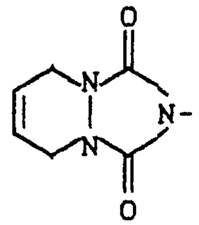 ;

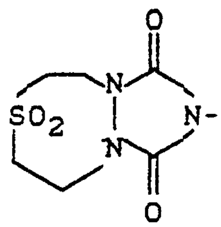 ; 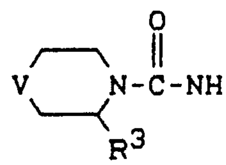 ; 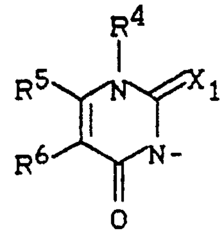 ;

oder 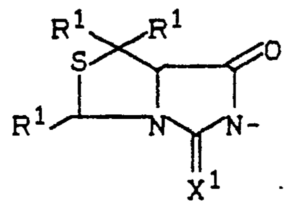 .

steht und

Q, Y, G, V, W, $R^1$, $R^2$, $R^3$, $R^4$, $R^5$, $R^6$, $X^1$ und $X^2$ die oben angegebene Bedeutung haben;

(E-α) wenn man Iso(thio)cyanate der Formel (VI)

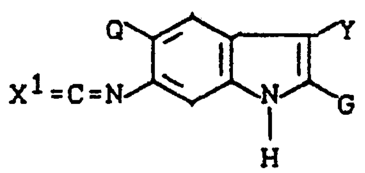

(VI)

in welcher

Q, Y und G die oben angegebene Bedeutung haben,

oder

(E-β) wenn man Carbamate der Formel (VII)

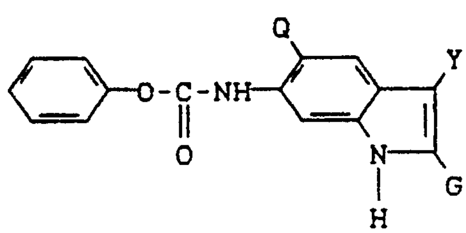

(VII)

in welcher

Q, Y und G die oben angegebene Bedeutung haben,

mit Carbonsäureestern der Formel (VIIIa)

$R^{13}$-$COOR^{14}$ (VIIIa)

in welcher

$R^{13}$ für einen Rest der Formel

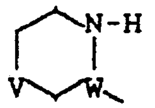 ; 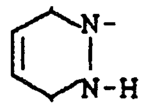 ; 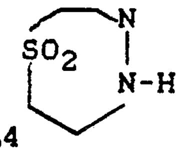 ;

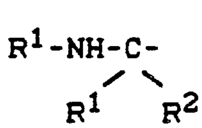 ; 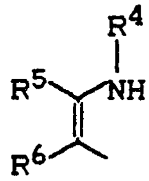 oder 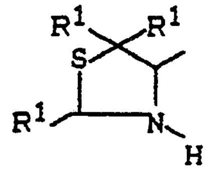 steht,

in welcher
$R^1$, $R^2$, $R^4$, $R^5$, $R^6$, V und W die oben angegebene Bedeutung haben und
$R^{14}$ für Alkyl steht,
oder mit Aminen der Formel (VIIIb)

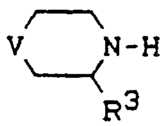 ; (VIIIb)

in welcher
V und $R^3$ die oben angegebene Bedeutung haben,
oder deren Säureadditionssalze,
gegebenenfalls in Gegenwart eines Verdünnungsmittels und gegebenenfalls in Gegenwart eines Reaktionshilfsmittels umsetzt;
(F) man erhält Heterocyclen der allgemeinen Formel (Ib-C)

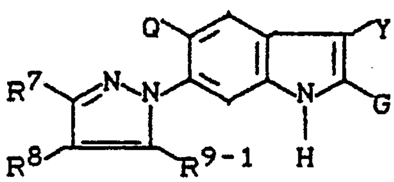 (Ib-C)

in welcher
$R^7$, $R^8$, Q, Y und G die oben angegebene Bedeutung haben und
$R^{9-1}$ für Wasserstoff, Alkyl oder Halogenalkyl steht,
wenn man Hydrazine der Formel (IX)

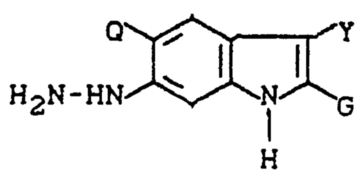 (IX)

in welcher
Q, Y und G die oben angegebene Bedeutung haben,
mit 1,3-Diketonen der Formel (X)

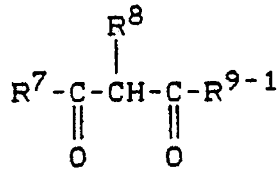

(X)

in welcher

$R^7$, $R^8$ und $R^{9-1}$ die oben angegebene Bedeutung haben,

oder mit Derivaten dieser Diketone, wie beispielsweise Enolethern, Enolestern, Ketalen, Enoletherketalen, Enaminen oder β-Halogenvinylketonen gegebenenfalls in Gegenwart eines Verdünnungsmittels und gegebenenfalls in Gegenwart eines Reaktionshilfsmittels umsetzt;

G) man erhält Heterocyclen der allgemeinen Formel (Ib-D)

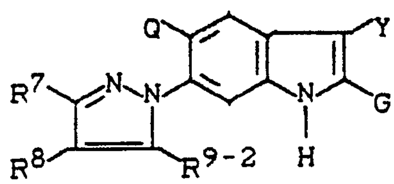

(Ib-D)

in welcher

$R^7$, $R^8$, Q, Y und G die oben angegebene Bedeutung haben und

$R^{9-2}$ für Halogen steht,

wenn man Pyrazolinyl-indole der Formel (XI)

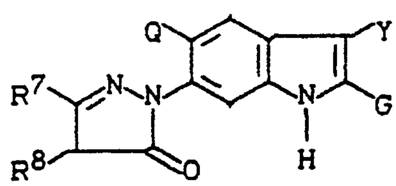

(XI)

in welcher

$R^7$, $R^8$, Q, Y und G die oben angegebene Bedeutung haben,

mit einem Halogenierungsmittel, gegebenenfalls in Gegenwart eines Verdünnungsmittels und gegebenenfalls in Gegenwart eines Reaktionshilfsmittels umsetzt;

H) man erhält Heterocyclen der allgemeinen Formel (Ib-E)

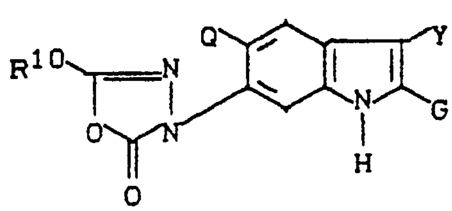

(Ib-E)

in welcher

$R^{10}$, Q, Y und G die oben angegebene Bedeutung haben,

wenn man Hydrazide der Formel

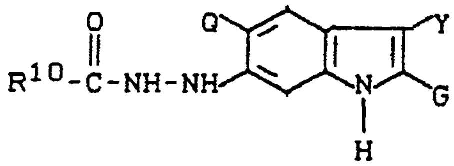

(XII)

in welcher

$R^{10}$, Q, Y und G die oben angegebene Bedeutung haben,

mit Phosgen gegebenenfalls in Gegenwart eines Verdünnungsmittels und gegebenenfalls in Gegenwart eines Reaktionshilfsmittels umsetzt;

I) man erhält Heterocyclen der allgemeinen Formel (Ib-F)

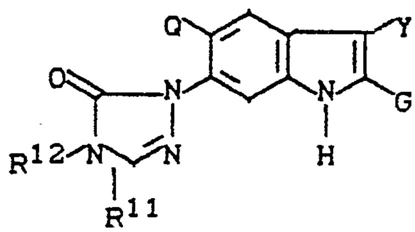

(Ib-F)

in welcher

$R^{11}$, $R^{12}$, Q, Y und G die oben angegebene Bedeutung haben,

wenn man Verbindungen der Formel (XIII)

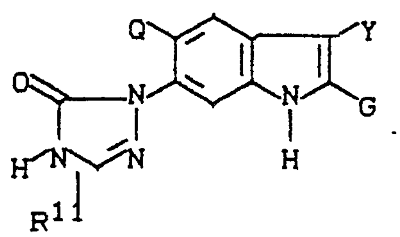

(XIII)

in welcher

$R^{11}$, Q, Y und G die oben angegebene Bedeutung haben,

mit Alkylierungsmitteln der Formel (XIV)

$R^{12}$-E (XIV)

in welcher

$R^{12}$ die oben angegebene Bedeutung hat und

E für eine elektronenanziehende Abgangsgruppe steht,

gegebenenfalls in Gegenwart eines Verdünnungsmittels und gegebenenfalls in Gegenwart eines Reaktianshilfsmittels umsetzt;

K) man erhält Heterocyclen der allgemeinen Formel (Ib-G)

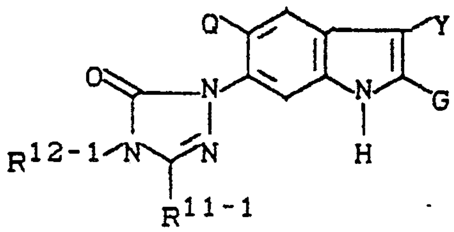

(Ib-G)

in welcher

Q, Y und G die oben angegebene Bedeutung haben und

$R^{12-1}$ und $R^{11-1}$ gemeinsam für einen zweifach verknüpften Alkandiylrest stehen, wenn man Amidrazone der Formel (XV)

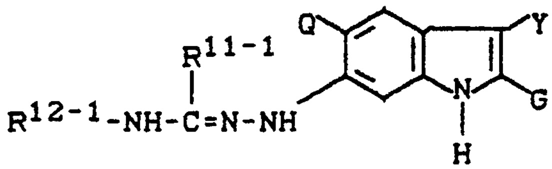

(XV)

in welcher

$R^{11-1}$ und $R^{12-1}$ die oben angegebene Bedeutung haben,

mit Phosgen gegebenenfalls in Gegenwart eines Verdünnungsmittels und gegebenenfalls in Gegenwart eines Reaktionshilfsmittels umsetzt;

L) man erhält Heterocyclen der allgemeinen Formel (Ib-H)

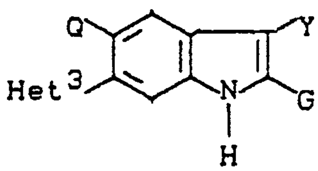

(Ib-H)

in welcher

$Het^3$ für einen Heterocyclus der Formel

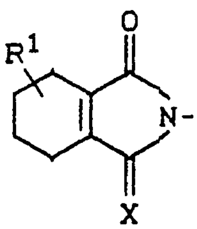 ; 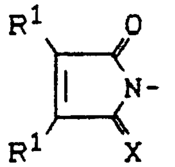 ; 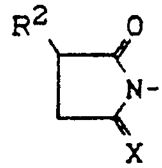 ;

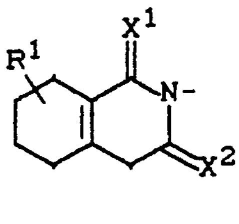 ; 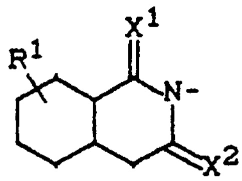 ; 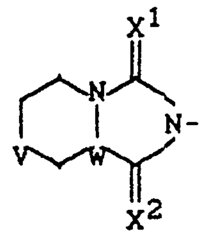 ;

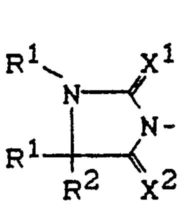 ; 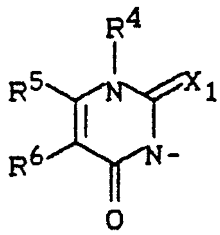 oder 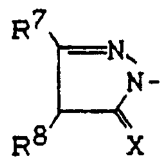

steht und

Q, Y, G, $R^1$, $R^2$, $R^4$, $R^5$, $R^6$, $R^7$, $R^8$, X, $X^1$, $X^2$, V und W die oben angegebene Bedeutung haben,

wobei

X für Schwefel und $X^1$ und $X^2$ jeweils für Sauerstoff oder Schwefel stehen, mit der Bedingung, daß mindestens einer der Reste $X^1$ oder $X^2$ für Schwefel steht;

(L-α) wenn man die mit Hilfe der Verfahren (D) und (E) erhältlichen Heterocyclen der Formel (Ib-1)

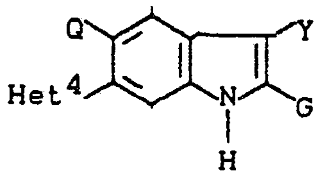

(Ib-I)

in welcher
Het[4] für einen Heterocyclus der Formel

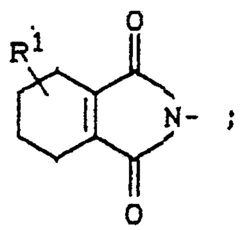
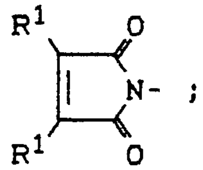
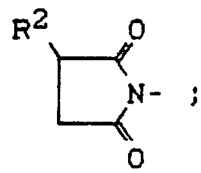
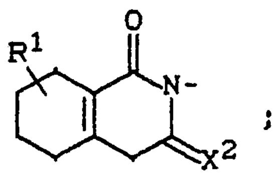
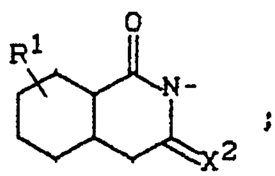
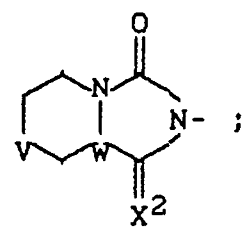
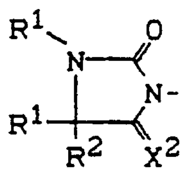

oder

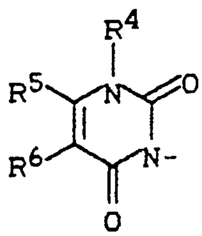

steht und
Q, Y, G, $R^1$, $R^2$, $R^4$, $R^5$, $R^6$, V, W und $X^2$ die oben angegebene Bedeutung haben;
oder
(L-β) wenn man Pyrazolinyl-indole der Formel (XI)

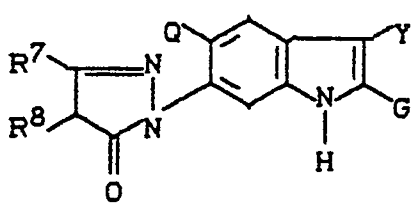

(XI)

in welcher
$R^7$, $R^8$, Q, Y und G die oben angegebene Bedeutung haben,
mit "Lawessons-Reagenz" der Formel (XVI)

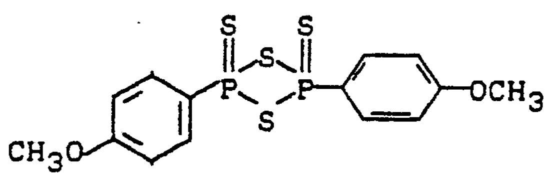

(XVI)

gegebenenfalls in Gegenwart eines Verdünnungsmittels umsetzt.

4. Herbizide Mittel, gekennzeichnet durch einen Gehalt an mindestens einem N-[Indol-6-yl]-heterocyclus der Formel (I) gemäß Anspruch 1 oder 3.

5. Verfahren zur Bekämpfung von unerwünschten Pflanzen, dadurch gekennzeichnet, daß man N-[Indol-6-yl]-heterocyclen der Formel (I) gemäß Anspruch 1 oder 3 auf die Pflanzen und/oder ihren Lebensraum einwirken läßt.

6. Verwendung von N-[Indol-6-yl]-heterocyclen der Formel (I) gemäß Anspruch 1 oder 3 zur Bekämpfung von unerwünschten Pflanzen.

7. Verfahren zur Herstellung von herbiziden Mitteln, dadurch gekennzeichnet, daß man N-[Indol-6-yl]-heterocyclen der Formel (I) gemäß Anspruch 1 oder 3 mit Streckmitteln und/oder oberflächenaktiven Substanzen vermischt.

8. Pyrazolinyl-indole der Formel (XI)

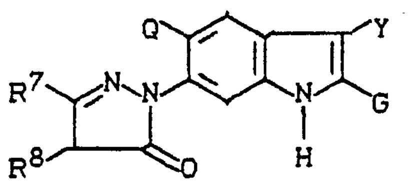

(XI)

in welcher

$R^7$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht,

$R^8$ für Wasserstoff, Alkyl mit 1 bis 8 Kohlenstoffatomen oder Halogenalkyl mit 1 bis 8 Kohlenstoffatomen und 1 bis 17 gleichen oder verschiedenen Halogenatomen steht, oder

$R^7$ und $R^8$ gemeinsam oder $R^8$ und $R^9$ gemeinsam für eine Alkylkette mit 4 bis 6 Kohlenstoffatomen stehen,

Q für Wasserstoff oder Halogen steht,

Y für Wasserstoff, Alkyl oder Halogen steht und

G für Wasserstoff oder Alkyl steht.

9. Hydrazide der Formel (XII)

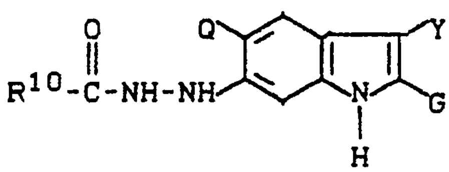

(XII)

in welcher

$R^{10}$ für Wasserstoff, Alkyl mit 1 bis 8 Kohlenstoffatomen, Halogenalkyl mit 1 bis 8 Kohlenstoffatomen und 1 bis 17 gleichen oder verschiedenen Halogenatomen oder unsubstituiertes oder substituiertes Cycloalkyl mit 3 bis 7 Kohlenstoffatomen steht,

Q für Wasserstoff oder Halogen steht,

Y für Wasserstoff, Alkyl oder Halogen steht und

G für Wasserstoff oder Alkyl steht.

10. Hydrazine der Formel (IX)

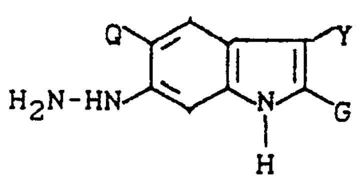

(IX)

in welcher

Q für Wasserstoff oder Halogen steht,

Y für Wasserstoff, Alkyl oder Halogen steht und

G für Wasserstoff oder Alkyl steht.